Europäisches Patentamt

European Patent Office

Office européen des brevets

(19)

(11) **EP 1 712 634 A1**

(12) **EUROPEAN PATENT APPLICATION**

(43) Date of publication:
**18.10.2006 Bulletin 2006/42**

(51) Int Cl.:
*C12Q 1/00* (2006.01)  *G01N 33/50* (2006.01)
*A61K 45/00* (2006.01)

(21) Application number: **05290815.9**

(22) Date of filing: **13.04.2005**

(84) Designated Contracting States:
**AT BE BG CH CY CZ DE DK EE ES FI FR GB GR HU IE IS IT LI LT LU MC NL PL PT RO SE SI SK TR**
Designated Extension States:
**AL BA HR LV MK YU**

(71) Applicant: **Wittycell SAS**
**51688 Reims Cedex 2 (FR)**

(72) Inventors:
• **Bernard, Jacky**
  **51500 Champfleury (FR)**
• **Millard, Anne-Laure**
  **51100 Reims (FR)**

(74) Representative: **Becker, Philippe et al**
  **Cabinet Becker & Associés**
  **35, rue des Mathurins**
  **75008 Paris (FR)**

(54) **Selection of highly efficient antigen presenting cells for regulating immunity and uses thereof**

(57)    The present invention relates, generally, to the fields of immunology and cellular biology. The invention discloses, more particularly, a new strategy of modulation of immune response based on the use of highly efficient non autologous antigen presenting and/or immunostimulatory system such as allogeneic antigen presenting cells. Invention thus relates to methods for preparing or selecting systems having high antigen presentation and/or immunostimulatory capacity for a given subject. The invention also discloses banks usable to select such systems as well as compositions comprising such systems, their preparation and uses.

EP 1 712 634 A1

**Description**

The present invention relates to novel compositions and methods for the modulation of immune responses in a subject. The invention more particularly relates to methods for the identification and/or characterization of non autologous antigen presenting and/or immunomodulatory systems, in particular allogeneic antigen presenting cells (APC) and their uses for preventive or curative therapy in mammalians, including human subjects. The invention also relates to particular methods for optimising an antigen preparation, as well as to methods of selecting subjects that respond to immunotherapy. The invention also discloses cell banks suitable to select non autologous antigen presenting and/or immunomodulatory systems, in particular allogeneic APC, as well as compositions comprising such systems, their preparation and uses. The present invention stems from the unexpected discovery that huge differences in the capacity to activate immune effector cells from a given subject exist between antigen presenting cells prepared from various donors. This discovery results in the possibility to select highly efficient or high performing non autologous antigen presenting and/or immunomodulatory systems for any given subject, allowing highly efficient immune modulation and highly efficient vaccination approaches to be developed. The invention may also -be used to modulate any type of immune response in any patient, and thus represents a novel avenue in the treatment of diseases in human subjects.

BACKGROUND OF THE INVENTION

Substantial progress has been made in vaccine development in recent years for the treatment of mammalian diseases, and in particular human diseases. New technologies have fostered the identification of potentially immunogenic antigens that can be used to activate a subject's immune system to specifically recognize and destroy target cells. Still a matter of intensive debate is the question of the most optimal system or condition to present antigens to the immune system to achieve the best response. In this area, major progress has been made by using dendritic cell (DC) based vaccines for stimulating immune responses against tumor antigens. The generation of new tools such as HLA-tetramer complexes now allows researchers to monitor more closely the expansion of peptide-specific T cells under the process of vaccination. Safety and immunogenicity of cultured autologous dendritic cells have been widely tested and confirmed in numerous clinical trials and particularly Bhardwaj et al demonstrate in healthy volunteers that a single injection of mature dendritic cells results in a rapid generation of broad T-cell immunity (Bhardwaj et al, J. Clin. Invest. 199 104 : 173-80). These conventional autologous vaccination strategies require complex and costly ex vivo manipulations of the patient's cells. Even if this approach suggests that tumour-specific immune responses can be achieved, it has resulted in an unsatisfactory small number of complete and durable tumour regressions in human, leading so far to disappointing outcomes. (Schultze JL et al, Trends Immunol. 2004 Dec;25(12):659-64). Although the interaction between transplantation and tumor immunology has been discussed in the past, the adjuvant potential of this interaction in anti-tumor and anti-viral immunization has hardly been evaluated so far. However, there are some potential advantages that alloreactivity could be used as a generic tool for generating immunostimulatory environment that enhances destructive anti-tumor immune responses. Indeed, unprimed T lymphocytes from one individual react with unusual vigour against the major histocompatibility complex (MHC) antigens of other members of the same species. Whereas precursor T-cell frequencies for normal environmental antigens (for example, virus proteins) are of the order of 1 in $1 \times 10^4$ or 1 in $1 \times 10^5$, approximately 1-10% of an individual's T lymphocytes will respond to the foreign MHC antigens of another individual. This phenomenon is a consequence of a unique aspect of T-cell biology: the capacity of T lymphocytes to recognize non autologous MHC molecules as intact structures on the surface of foreign cells.

The extraordinarily high precursor frequency for direct T cell non autologous recognition has three broad implications for the generation of these self MHC restricted T-cell responses.

-    First, the vast numbers of T cells responding by direct recognition to an intact non autologous MHC molecule will also have specificity for normal environmental peptides presented by self MHC molecules.
-    The second implication concerns harnessing the potent CD4+ T-cell direct non autologous recognition response to MHC class II molecules for provision of T-cell help for the self-restricted responses to tumor peptides. Because the provision of CD4+ T-cell help in tumors is likely to be weak or absent, this is a critical consideration. Not only does the direct non autologous recognition pathway bypass the need for presentation of tumor peptides by self MHC class II molecules but, because of the high precursor T-cell frequency, the help is unusually potent.
-    Third, the importance of the *innate* immune system for generating an immunostimulatory environment for effective immune responses is now well recognized. Non autologous, in particular allogeneic responses have the potential to provide a milieu rich in cytokines and T-cell costimulatory ligands for the promotion of T-cell responses (Fabre JW, 2001)

Considering the above potential advantages, allogeneic vaccination has surprisingly been tested in only very few trials. These trials further led to variable results. Lee et al. reports the generation of cytotoxic CD8 T lymphocytes using leukemic lysate pulsed donor cell-derived dendritic cells (Je-Jung Lee et al, 2004). Some articles also relate to uses of allogeneic DC to induce specific cytotoxic T lymphocytes responses in HIV-infected individuals without clinical benefit (Kundu SK et al, 1998). Moreover, Höltl L. et al. (2004), while evaluating the efficacy of allogeneic dendritic cells in the treatment of patients with metastatic renal cell carcinoma, concluded that "Allogeneic immunotherapy with DC is feasible and well tolerated. However, the immunogenicity of allogeneic DC is clearly less pronounced than that of autologous DC immunotherapy". Thus, even if allogeneic vaccination is regarded as an interesting alternative to autologous vaccination, negative results have been observed which led to the conclusion that this type of vaccination is, in the state of current knowledge, rather not very reproducible or foreseeable.

Millard et al. (CFCD, Paris, December 2002 and AACR, Toronto, April 2003) relate to the amplification of a specific T lymphocyte response using allogeneic DC but does not describe or suggest the existence of HLA haplotype-dependent and/or independent differences between individuals in antigen presentation or immune modulation, and do not provide any method to select or prepare high performance presentation systems. These documents do not either suggest methods of optimising antigen preparations nor any process for selecting or identifying responder patients.

Thus, there is a need in the art for compositions and methods to induce powerful immune responses which can be used as new immunotherapeutic tools in mammal.

SUMMARY OF THE INVENTION

The present invention now provides compositions and methods for safe and efficient non autologous modulation of immunity, preferably allogeneic vaccination, of mammals, particularly of human subjects. The present invention stems from the unexpected discovery that considerable differences in the capacities to activate immune responses from a given subject exist between APC from different individuals. The present invention is based on the use of high performance non autologous, preferably allogeneic, APC selected from various donors. Up to the present invention, allogeneic donor APCs were used in vaccination trials without taking this parameter into account. Up to the present invention, this parameter had simply never been considered and identified. The present invention now proposes and demonstrates that successful and highly efficient vaccination can be achieved using high-performance non autologous antigen presenting and/or immunomodulatory systems adapted to a subject. The invention shows that allogeneic APCs selected or prepared with a method according to the invention can lead to a logarithmic increase or reduction in both the NK and T cell response as compared to autologous APCs from the subject, thus revealing new prospects in the immune response levels likely to be obtained in immunotherapy via non autologous vaccination.

An aspect of the present invention thus resides in a method for modulating an immune response in a subject, particularly a human subject, using a high performance non autologous antigen presenting and/or immunomodulatory system, more preferably a high performance allogeneic APCs or a membrane material derived therefrom.

In a particular embodiment, the invention relates to a method for modulating an immune response to an antigen in a subject, the method comprising administering to the subject a composition comprising a high performance non-autologous non autologous antigen presenting and/or immunomodulatory system, or an immune cell activated by such a system, wherein said system causes in vitro an increase or a reduction in the subject T cell response at least 5 times greater than autologous antigen presenting cells from the subject, preferably at least 10 times, 20 times, 50 times or, even more preferably, at least 100 times, and wherein said system shares at least one MHC haplotype with said subject.

Another aspect of this invention lies in the use of a high performance non autologous antigen presenting and/or immunomodulatory system, preferably allogeneic APCs or a membrane material derived therefrom, for the manufacture of a pharmaceutical composition for the modulation of an immune response in a subject, particularly a human subject, wherein said system preferably causes in vitro an increase or a reduction in the subject T cell response at least 5 times greater than autologous antigen presenting cells from the subject, preferably at least 10 times, 20 times, 50 times or, even more preferably, at least 100 times, and wherein said system shares at least one MHC haplotype with said subject.

The immune response that is to be modulated in said subject can be specific for an antigen or can be a non specific immune response. It is preferably an antigen-specific immune response. In the latter situation, the composition comprises said allogeneic APCs or the membrane material derived therefrom and said antigen.

A further aspect of this invention is a pharmaceutical composition for modulating an immune response in a host subject, comprising a non autologous antigen presenting and/or immunomodulatory system or an immune cell activated by such a system and a pharmaceutically acceptable excipient or diluent, wherein said system preferably causes in vitro an increase or a reduction in the host subject T cell response at least 5 times greater than autologous antigen presenting cells from said subject, preferably at least 10 times, 20 times, 50 times or, even more preferably, at least 100 times, and wherein said system shares at least one MHC haplotype with said subject. In a particular embodiment, the non-autologous system comprises a selected antigen.

Another aspect of the invention relates to methods for selecting non autologous antigen presenting and/or immunomodulatory systems, such as allogeneic APCs, for treatment of a given subject. The non-autologous systems are typically selected by a method comprising:

- assessing the antigen presentation and/or immunomodulatory capacity of at least two non autologous antigen presenting and/or immunomodulatory systems towards white blood cells from a host subject, and
- selecting non autologous antigen presenting and/or immunomodulatory systems having a significant difference in antigen presentation and/or immunomodulatory capacity for said host subject as compared to autologous antigen presenting cells from said host subject.

As will be disclosed further below, the systems may be selected by various approaches, including by biological assay(s) or by phenotypic analysis.

The invention further relates to a bank, wherein the bank comprises a plurality of non-autologous, allele-phenotyped antigen presenting and/or immunomodulatory systems (such as APCs), preferably from different human donors, each of which being comprised in at least one distinct system or cell storage unit for storage of cellular material.

The invention also relates to a method for preparing a composition for modulating an immune response in a subject, the method comprising:

(a) providing a library of non autologous antigen presenting and/or immunomodulatory systems,
(b) assessing the antigen presentation and/or immunomodulatory capacity of systems from the library towards white blood cells from the subject,
(c) selecting systems from said library having a significant difference in antigen presentation and/or immunomodulatory capacity for said host subject as compared to autologous antigen presenting cells from said host subject, and
(d) preparing a composition using systems selected in step (c). Step (d) may include a step of loading the selected systems with an antigen or a nucleic acid encoding an antigen.

Another aspect of the invention relates to a method for optimizing an antigen preparation by comparing an immune response to said antigen preparation and to one or several variant antigen preparations thereof, wherein the immune response is determined by contacting said antigen preparations with at least one non autologous antigen presenting and/or immunomodulatory system selected by a method according to the invention or from a bank according to the invention, and selecting variant antigen preparation(s) causing an optimized immune response. In a particular embodiment, the antigen preparations are contacted with at least two antigen presenting cells having a significant difference in antigen presentation capacity.

A further aspect of this invention is a method for selecting a host subject that responds to an immunotherapeutic treatment, comprising a step of contacting immune cells, preferably T cells, from said subject, in the presence of said immunotherapeutic treatment, with at least one non autologous antigen presenting and/or immunomodulatory system selected by a method according to the invention or from a bank according to the invention, and assessing the response of said immune cells, said response being indicative of a responder subject. In a particular embodiment, the T cells and treatment are contacted with at least two antigen presenting cells having a significant difference in antigen presentation capacity.

As will be further described, the invention can be used to modulate (e.g., increase, stimulate, cause, reduce, etc.) different types of immune responses in a subject, such as, without limitation, antigen-specific immune responses, innate immunity or suppressive immune response. The invention can be used to prevent or treat various diseases, including but not limited to tumor diseases, inflammatory diseases and infectious diseases, either alone or in combination with additional treatments.

LEGEND TO THE FIGURES

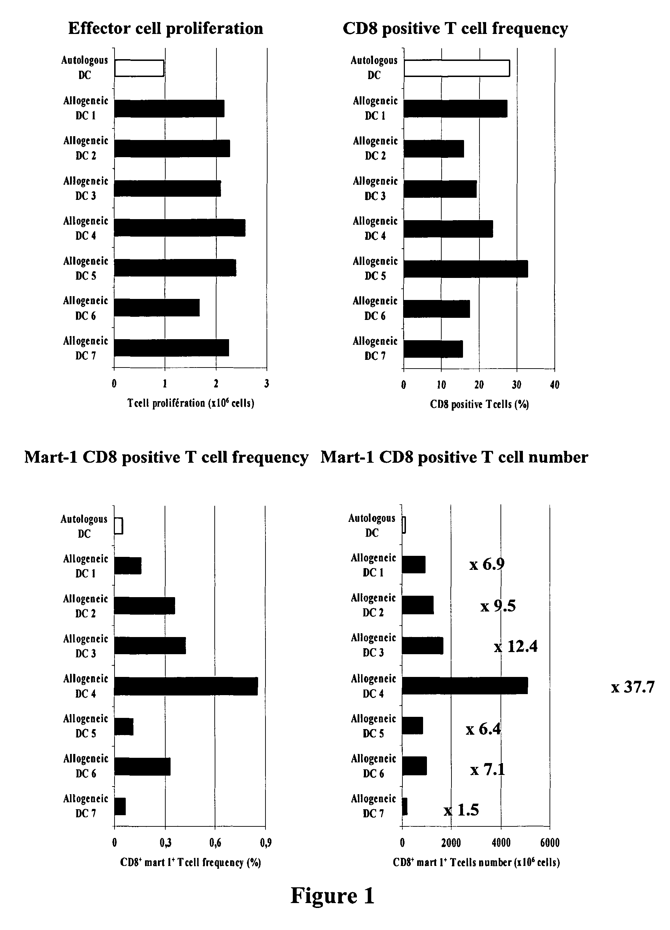

FIGURE. 1 is a bar graph in relation to example 1 employed to assess the possibility to select HP-APC that augment mart-1 specific CD8 positive T cell number. 7 random allogeneic DC (black bar) were compared to the autologous DC (white bar) for their ability to induce mart-1 specific T cell expansion. Clonal expansion was determined using specific tetramers.

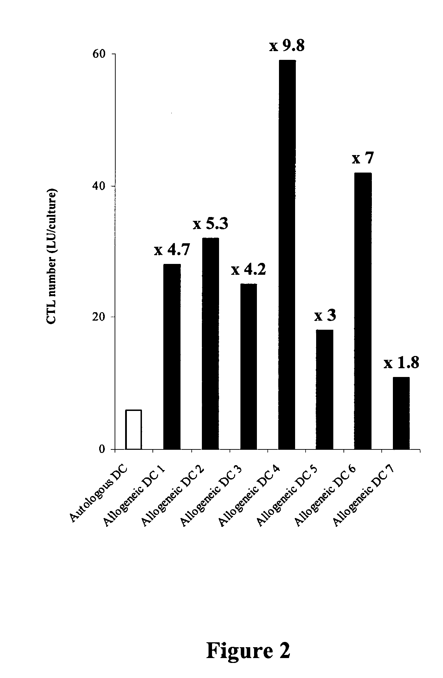

FIGURE. 2 is a bar graph in relation to example 2 employed to assess the possibility to select HP-APC that increase influenza M1 specific CTL number. 7 random allogeneic DC (black bar) were compared to the autologous DC (white bar) for their ability to induce influenza M1 specific CTL. Specific lytic activity was determined using standard chromium release assay.

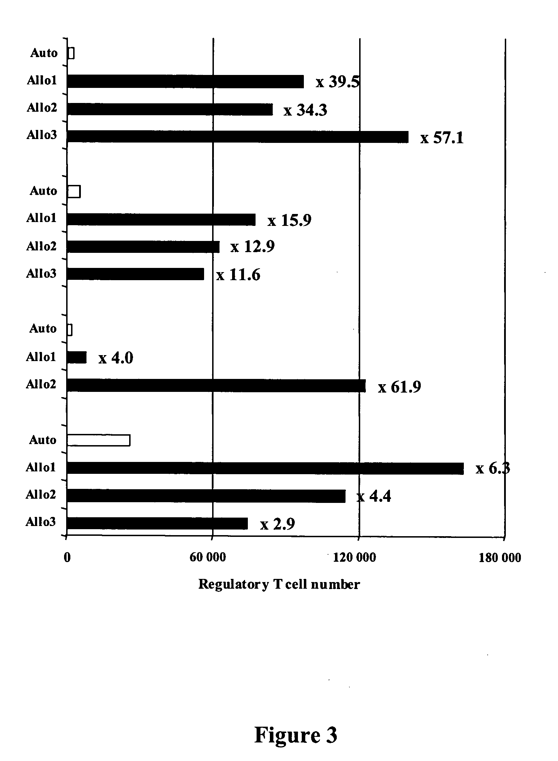

FIGURE. 3 is a bar graph in relation to example 3 employed to assess the possibility to select HP-APC that amplify regulatory T cell differentiation. 3 random allogeneic DC (black bar) were compared to the autologous DC (white bar) from 4 different donors for their ability to induce regulatory T cell differentiation. Regulatory T cell expansion was determined following staining with antibodies to human CD4, CD25 and CTL-A4 (or CD152) and analyzed in an EPICS XL flow-cytometer.

FIGURE. 4

4A and 4B are bar graphs in relation to example 4 employed to assess the possibility to select HP-APC that enhance NK cell proliferation. 5 random allogeneic DC (black bar) were compared to the autologous DC (white bar) for their ability to enhance CD3-CD56$^+$ cell proliferation. CD3-CD56$^+$ cell proliferation was determined using 5- (and 6-) carboxyfluorescein diacetate succinimidyl ester, following staining with antibodies to human CD3 and CD56 and analyzed in an EPICS XL flow-cytometer. 4A reflects a pourcentage of dividing cells and 4B a cell division number.

FIGURE. 5

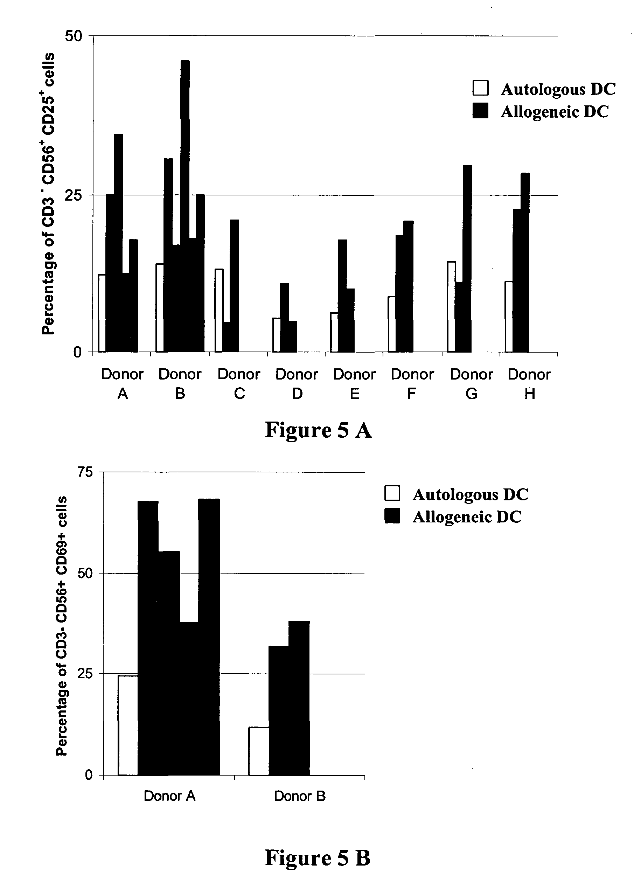

5A and 5B are bar graphs in relation to example 5 employed to assess the possibility to select HP-APC that enhance NK cell activation. 2 to 5 random allogeneic DC (black bar) were compared to the autologous DC (white bar) from 8 different donors for their ability to enhance CD3-CD56$^+$ cell activation. CD3-CD56$^+$ cell activation was determined following staining with antibodies to human CD3, CD56 and CD69 (Fig. 5B) or human CD3, CD25 and CD56 (Fig. 5A) and analyzed in an EPICS XL flow-cytometer.

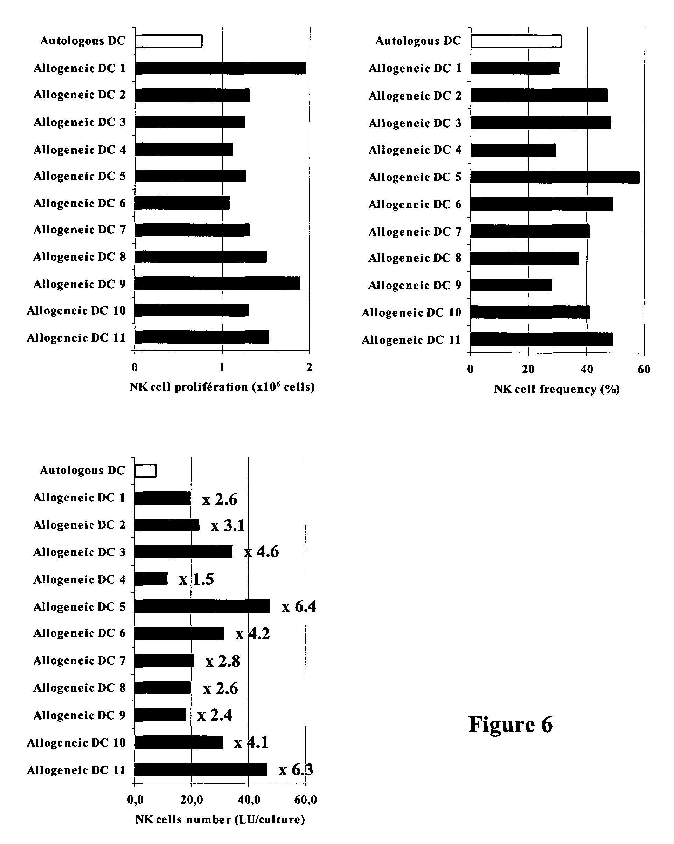

FIGURE. 6 is a bar graph in relation to example 6 employed to assess the possibility to select HP-APC that enhance NK cell effector function. 11 random allogeneic DC (black bar) were compared to the autologous DC (white bar) for their ability to increase NK cell effector function. NK cell lytic activity was determined using standard chromium release assay.

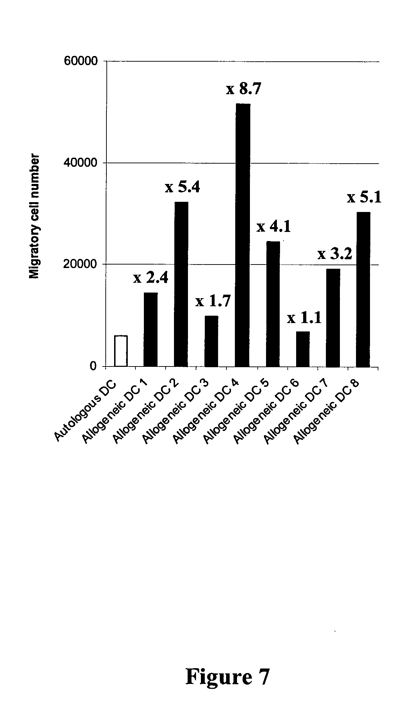

FIGURE. 7 is a bar graph in relation to example 7 employed to assess the possibility to select HP-APC with enhanced migratory capacity. 8 random allogeneic DC (black bar) were compared to the autologous DC (white bar) from a given healthy volunteers for their migratory capacity. Cell migration was determined using a conventional transwell™ system.

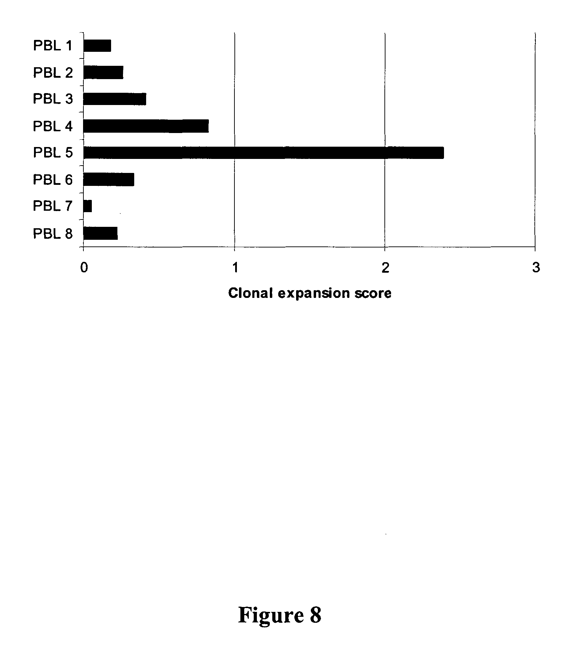

FIGURE. 8 is a bar graph in relation to example 8 employed to assess donor sensibility to immunotherapy., A panel of 3 HP-APC were used to present mart-1 peptide to lymphocytes from 8 different healthy volunteers. Mart-1 specific clonal expansion was determined using specific tetramers.

DETAILED DESCRIPTION OF THE INVENTION

As indicated above, the present invention aims to use non autologous reactivity, preferably alloreactivity, as a generic tool for generating an immunostimulatory environment. The present invention is more specifically based on the use of high performance non autologous antigen presenting and/or immunomodulatory systems, such as allogeneic antigen presenting cells (APC), as a source of adjuvant and/or as a vehicle to deliver antigens.

The present invention stems from the unexpected discovery that considerable differences in the capacities to activate immune responses from a given subject exist between APC from different individuals. The present invention is based on the use of high performance non autologous, preferably allogeneic, APC selected from various donors. The present invention thus relates to methods for preparing or selecting high performance non autologous antigen presenting and/or immunomodulatory systems, and to their uses for vaccination of a given subject. The invention also relates to compositions comprising such systems, their preparation and uses.

As disclosed in the present application, this novel approach allows to increase both the self MHC-restricted response to antigens and NK cell activity against target cells.

**Production of non autologous antigen presenting and/or immunomodulatory systems**

A first object of the invention relates to methods for producing or selecting high performance non autologous antigen presenting and/or immunomodulatory systems. The methods typically comprise:

- assessing the antigen presentation and/or immunomodulatory capacity of at least two non autologous antigen presenting and/or immunomodulatory systems, towards white blood cells from a host subject, and
- selecting a non-autologous antigen presenting and/or immunomodulatory system having a significant difference in antigen presentation and/or immunomodulatory capacity for said host subject as compared to autologous antigen presenting cells from said host subject.

The antigen presentation and/or immunomodulatory capacity of at least two non-autologous antigen presenting and/or immunomodulatory systems is assessed towards white blood cells from a host subject. In a preferred embodiment, the first step of the above described method is performed using at least 3, 4, 5, 8, 10, 12, 16, 20 or 50 distinct non autologous antigen presenting and/or immunomodulatory systems. The selected system(s) has(ve) a significant difference in antigen presentation and/or immunomodulatory capacity for said host subject as compared to autologous antigen presenting cells from said host subject. The difference may be either favourable or unfavourable to antigen presentation and/or immunomodulation. Such system will then be usable to amplify or inhibit an immune response in said particular host subject.

As will be disclosed therein, there is virtually no limit as to the type of systems that can be used in a method according to the invention for selecting non autologous antigen presenting and/or immunomodulatory systems. Such systems may be selected for example from a mammalian cell, in particular a human cell, from a recombinant cell, a cell derivative, a liposome, a polymer, etc.

Cells may be, for example, immune or non immune cells, natural antigen presenting cells or artificially engineered antigen presenting cells.

Methods according to the invention are preferably used to produce or select non autologous high performance antigen presenting cells (HP-APCs).

Within the context of the present invention, non autologous high performance systems, preferably APCs (HP-APC), typically designate allogeneic systems /APCs that cause in vitro an increase or a reduction in a subject T cell response at least 5 times greater than autologous APCs from the subject, preferably at least 10 times, 20 times, 50 times or, even more preferably, at least 100 times, 1000 times, 10 000 times or 100 000 times. Even more preferably, the allogeneic high performance systems / APCs share at least one MHC haplotype with said subject, e.g., the MHC allele that is involved in antigen restriction.

Preferred cells used for selecting HP-APCs are immune cells, in particular natural antigen presenting cells, such as, without limitation, dendritic cells, monocytes, mast cells, cells of myeloid lineage, B cells, Langerhans cells, bone marrow cells, or precursors thereof, including CD34+ stem cells. Peripheral blood mononuclear cells (PBMC) may also be used. Dendritic Cells (DC) represent a preferred embodiment of this invention. DC are the most potent known antigen-presenting cells for initiating cellular immune responses through the stimulation of effector cells. Their action is due mainly to both expression of various molecules related to adhesion, MHC and costimulatory pathway and cytokine secretion (Banchereau J et al, Ann N Y Acad. Sci. 2003 Apr;987:180-7). In a particular embodiment, HP-APCs of this invention are thus HP-dendritic cells

Other cells that may be used to produce HP-APCs include, without limitation, fibroblasts, hepatocytes, keratinocytes, muscle cells or precursors thereof. These cells may be genetically modified to express molecules involved in antigen presentation, and thus behave as an APC.

Suitable cells for use in preparing HP-APCs according to the invention may be obtained from existing cell banks, from established, modified or transformed cell lines such as U-937, BDCM, etc., or may be directly collected from one or more donor subjects, in particular a mammal, preferably a human, preferably of the same species as the subject which is to be treated. In a preferred embodiment, cells are collected from healthy subjects. Cells from non essential tissues that are irrelevant to the host may also be appropriate as they reduce the risk of induction of autoimmune disease. As indicated above, such cells may be genetically modified, loaded or pulsed to present a desired antigen, preferably a disease-specific antigen, and/or any costimulatory molecule.

In a particular embodiment, the HP-APCs may be produced or selected from a library of mammalian cells comprising cells from distinct donors, which may be stored under appropriate conditions (e.g., into separate and identifiable containers) and, preferably, at least partially phenotyped.

In this regard, the invention also relates to a bank, wherein the bank comprises a plurality of non autologous antigen presenting and/or immunomodulatory systems, in particular non-autologous antigen presenting and/or immunomodulatory cells from different donors, preferably from different human donors, each of which being comprised in at least one distinct system or cell storage unit for storage of cellular material. Such banks allow the rapid and efficient selection of high performance systems, preferably HP-APCs, for use in treating any subject.

Antigen presenting and/or immunomodulatory systems are preferably allele-phenotyped, in particular HLA-phenotyped.

In a particular embodiment, the invention relates to a bank, as described above, comprising antigen presenting cells having alleles selected from major HLA, such as any Class I, II or III HLA, minor HLA, HLA G and non polymorphic alleles, such as any member of the CD1 family members for example.

Selection of Donors

The term "donor" refers to any mammalian organism from whom cells are collected, said cells being intended for the preparation of a bank according to the invention or for future treatment of a subject by non autologous, preferably allogeneic vaccination, optionally via particular pharmaceutical composition(s) according to the invention.

The present invention applies to cells collected from any mammalian, in particular from a human subject. An example of such a mammal is a human such as a human infant, child, or adult. For ease of discussion in this patent application, we use a human being (identified as a person or a donor) as a nonlimiting example of such an mammalian subject.

Prior to being used in a method of this invention, a donor may be subjected to a medical examination to confirm his health status. Healthy donors are mostly preferred, although, under certain circumstances, a donor in a "pre-disease" or "disease" state with respect to a specific disease may not be excluded. At the time of APC collection, the donor may be diagnosed to have a disease or diseases, or class or classes of diseases different from the specific disease, which diagnosed diseases may be acceptable with respect to APC collection and/or with respect to the specific disease as perceived by prevailing medical practices.

Donor selection standards may include one or more of the following considerations prior to collection, such as (a) pre-specific disease; (b) specific or general diseases; (c) parameters of the donor relating to certain diseases, for example a certain age, certain physical conditions and/or symptoms, with respect to certain specific diseases, with respect to certain prior treatment history and/or preventive treatment, etc.; (d) whether the donor fits into one or more established statistical and/or demographic models or profiles (e.g., statistically unlikely to acquire certain diseases); and (e) whether the donor is in a certain acceptable health condition as perceived based on prevailing medical practices, etc.

Cells may be selected using a buffy coat source or by apheresis for example.

Cells are preferably collected by apheresis from donor's peripheral blood, processed (to optimise the quantity and quality of the collected cells) and, optionally cryogenically preserved or maintained in culture under suitable conditions.

Depending on the situation and the quantity and quality of cells to be collected from the donor, it may be preferable to collect the cells from donors when they are at an "adult" or a "matured" age (the term "adult" as used herein refers to and includes adult and non-neonate, unless otherwise used in a particular context to take a different meaning) and/or at a certain minimum weight. For example, the cells are collected when the donor is within a range from 10 to 200 Kg or any sub-range thereof, such as 20 to 40 Kg. In addition or in the alternative, it may be required that the donor be of a certain age, e.g., within a range from 2-80 years old or any sub-range thereof such as 9 to 18 years old, or 12 to 16 years old. Certain legal requirements may also prescribe and/or limit the appropriate age and/or or weight of the donor for cell collection.

Collection, Purification And Storage

The physical steps of collecting cells may comprise steps known per se in the art.

Blood cells may be collected using a continuous-flow cell separator such as Spectra (COBE laboratory, Lakewood, CO). Many hundreds of thousands of apheresis collections take place each year for platelets, red cells, plasma and stem cells. This procedure has been shown to be safe and effective. Also, if required, the amount of cells circulating in the peripheral blood cells may be increased with the infusion of cell growth factors prior to collection.

Cell collection can be performed in any place, such as in a collection centre. Typically, blood is drawn from one arm and then enters the apheresis instrument where the desired cells are separated and collected. The rest of the whole blood is then returned to the donor. Shortly after apheresis collection, the bone marrow releases more stem cells into the blood stream to replace the harvested cells. The amount of blood cells collected is a very small fraction of a subject's blood cells so that the procedure does not deplete the body of its blood cells.

Cell purification can be performed by countercurrent centrifugal elutriation. Elutriation may be performed by stepwise increments of the flow rate. Size and concentration of elutriated cells are preferably closely monitored by flow cytometry (cf. De Carvalho CM et al, 2004 ; Pandita TK, 2004).

Affinity separation may also be used to isolate donor cells from any biological sample (tissue, biological fluid, etc.), using for instance protein-coated magnetic particles or other types of solid supports such as polystyrene particles. Such affinity separation protocol is well known in the art. Various methods for sorting biological populations via affinity separations on solid supports or using immunorosettes have been described in the literature [see for instance US3,970,518, US4,710,472 US6,872,567; Bernan et al., J. Immunol., 138: 2100-03 (1987)]. In performing such methods, a binding molecule (e.g., monoclonal antibody) is typically conjugated to the solid supports such as the magnetic particles or plastic beads, and added to a test sample under conditions causing binding to a characteristic determinant on the analyte of interest. The cells complexed with the solid support are then separated from the uncomplexed cells by exposure to a magnetic field or filtration or other method depending on the nature of the solid support. These techniques may also be used to separate lymphocytes subpopulations.

Following collection and/or purification, the cells can be placed in any container, which may be sealed and then transported to the laboratory for processing, testing, phenotyping and/or (cryo)preservation. Cells may be transported by methods known in the art. For example, conventional containers for blood or other biological fluids can be used.

Upon collection, the cells can be processed to improve the quality of a bank, in particular a cell library, of this invention. More specifically, such processing may include the following steps: preparation of containers (e.g., tubes) and labels, sampling and/or testing of the collected material, centrifugation, transfer of material from collection containers to storage containers, the addition of cryoprotectant, phenotyping of the cells, etc.

The physical steps of cell storage may include the use of cryo-protectant (DMSO), controlled rate freezing and storage within a liquid nitrogen filled tank.

The collected cells can be aliquoted into defined dosage fractions.

The cells may be stored under any appropriate conditions, such as in culture or in a frozen or lyophilized state. Cryopreservation can be obtained using a variety of cryoprotecting agents, such as DMSO.

Phenotyping

In a most preferred embodiment, the systems, in particular the cells, for use in the present invention are phenotyped, at least with respect to certain parameters, such as for instance a cell surface marker or a HLA haplotype. This typing may be based on genotypic or phenotypic criteria as discussed below. This phenotyping facilitates the production of high performing non autologous antigen presenting and/or immunomodulatory systems, HP-APCs for example, as well as their in vivo uses.

In a preferred embodiment, the systems are phenotyped with respect to at least one identified characteristic reflecting a particular information. The phenotypic information or trait may include any observable or measurable characteristic, either at a macroscopic or system level or microscopic, cellular or molecular level.

Phenotyped APCs subsets of interest may for example be distinguished by expression of characteristic marker or cell surface determinants and/or secretion of (functional-related) molecules. In addition, cells from the same subset but at different stages of differentiation may be distinguished by expression of other particular characteristic cell surface determinants. Cells with different functional activity or at different times after an initial interaction with a mitogen or antigen can also express different cell surface determinants or secrete different (functional-related) molecules. The determinant (s) may thus be specific for a functional, differentiation, or activation marker on the cell surface. They may, in particular, reflect a particular function of the cell carrying them, preferably a function in relation with the immunomodulatory capacity , i.e., the capacity to modulate an immune response (e.g., proliferation of white blood cells or subsets thereof). The determinant(s) may also be specific for a diseased status, such as for example an infectious status.

When used in reference to the methods of the invention, "High Performing (HP) or Highly Efficient (HE)-associated determinant" means any molecule expressed inside a cell or on the cell surface, that identifies a HP or HE cell. HP-associated determinants include, for example, components of the cell membrane, such as membrane bound proteins or glycoproteins or lipids or glycolipids and including cell surface antigens, histocompatibilty antigens, or membrane receptors. They may also include adhesion/coactivation molecule(s).

The marker or HP-associated determinant may preferably be any molecule implicated in the immunological process (Friedl. P. et al., 2004). Particular examples of such a marker or HP-associated determinant may be selected from the B7 family members, which regulate T cell activation and tolerance (Greenwald RJ et al., 2005), the TLR family members, the TNF receptor family members, CD83, Chemokines (such as CCR7), integrins, CD40, CD223, etc.

Genotypic information may refer to a specific genetic composition of a specific individual organism, including one or more variations or mutations in the genetic composition of the individual's genome and the possible relationship of that genetic composition to disease. An example of this genotypic information is the genetic "fingerprint" and the Human Leukocyte Antigen (HLA) type of the donor.

In a preferred embodiment, systems / APCs are allele-phenotyped, preferably HLA-allele phenotyped. Polymorphic alleles can for example be selected from major HLA, minor HLA and HLA G. Major HLA alleles may more specifically be selected from any class I HLA such as HLA-A1, HLA-A2, HLA-A3, HLA-A24, HLA-A11, HLA-A28, HLA-A29, HLA-A32, HLA-B15, HLA-B5, HLA-B7, HLA-B8, HLA-B12, HLA-B14, HLA-B18, HLA-B35, HLA-B40, HLA-C group 1, HLA-C group 2 for example, any class II HLA-DPB9, HLA-DPB11, HLA-DPB35, HLA-DPB55, HLA-DPB56, HLA-DPB69 HLA-DPB84 HLA-DPB 87, HLA-DRB1, HLA-DQA1, HLA-DQB1, or any class III HLA. The knowledge of a HLA phenotype can facilitate subsequent selection of HP-APCs, as will be discussed below.

Systems / APCs may also be phenotyped with a non polymorphic allele such as alleles selected for example from any member of the CD1 family members (CD1 a, CD1b, CD1 c, CD1 d, CD1 e).

Methods of determining HLA transcription or expression profile of a cell are known per se in the art (see for instance WO99/42128) and may be used to prepare HLA-phenotyped cell banks of this invention.

The phenotype of a cell may also be determined with respect to determinants on the cell surface and/or the presence of or the reactivity towards specific binding substances, such as lectins, hormones, cytokines, receptor ligands, etc.

Genetic Modification of the Cells

In addition, cells for use in (or in a cell bank according to) the invention may contain a recombinant nucleic acid comprising one or several (e.g., two or more) open reading frames encoding any molecule of interest, such as for instance an antigen, a co-stimulatory molecule or a presentation molecule (e.g., a MHC molecule). Such recombinant nucleic acids may be isolated and purified free from other nucleotide sequences by conventional purification techniques, e.g., using restriction enzymes to isolate desired fragments. The nucleic acid may also be synthesized in vitro, using standard methodology. A recombinant nucleic acid for use in the context of the present invention may be selected from DNA (in particular cDNA) or RNA, and may further comprise regulatory sequences (e.g., promoter, terminator, polyA, etc., to ensure proper expression), as well as vector sequences.

Any of the techniques which are available in the art may be used to introduce a recombinant nucleic acid into the allogeneic cell. These techniques are collectively referred to herein as transfection and include, without limitation, transfection with naked or encapsulated nucleic acids, cell fusion, protoplast fusion, viral infection, cellular endocytosis of calcium-nucleic acid microprecipitates, fusion with liposomes containing nucleic acids, and electroporation. The choice of suitable vectors for expression is well within the skill of the art. Any vector can be chosen by the practitioner for particularly desirable properties, such as plasmids, viruses, episomes, artificial chromosomes and the like. Expression of the encoded polypeptide may be determined by any of a variety of methods known in the art, such as immunocytochemistry, ELISA, Western blotting, radioimmunoassay, or protein fingerprinting.

Banking

The collected and processed systems, in particular cells or part thereof, are preferably "banked" for future use, at a cell bank or depository or storage facility, or any place where systems such as cells are kept for safekeeping. Furthermore, appropriate computer systems can be used for data processing, to maintain records relating to donor information and to ensure rapid and efficient retrieval of cells from the storage repositories.

In a particular embodiment, each of the storage containers (e.g., bags or tubes) can be tagged with positive identification based on a distinctive property associated with the donor, lines or cell type, prior to storing in a bank according to the invention. For example, DNA genetic fingerprint and HLA typing may be used with secured identification mechanism such as acceptable methods using microchips, magnetic strip, and/or bar code labels. This identification step may be included in the banking process.

The systems are typically banked in separate storage units, preferably of defined dosages, each unit containing one distinct system or a pre-defined system number. At the time of use, only the required storage unit is retrieved, the number of units necessary to fulfill a desired dosage being selectable. Certain diseases may require cell therapy that includes a series of repeated treatments. By providing unitized storage of systems, only the required system and/or dosage may be retrieved for each treatment, to complete the entire therapy. The population of desired systems may also be extracted from the bank and increased in the case of cells by cellular expansion before preparation of the pharmaceutical composition and administration to the patient. The systems, in particular cells, may further be programmed by growing them in vitro under stimulation by desired chemicals or cytokines, or genetically modified as described previously. Prior to uses, the systems may also be grown with the subject's diseased cells or tissues, pulsed with an antigen, for example a known tumour-associated antigens, loaded with tumor extracts, or fused with diseased cells, for example tumor cells.

Determination of antigen presentation and/or immunomodulatory capacity

As indicated above, the method for selecting a non autologous antigen presenting and/or immunomodulatory system typically comprises:

- assessing the antigen presentation and/or immunomodulatory capacity of at least two non autologous antigen presenting and/or immunomodulatory systems towards white blood cells from a host subject, and
- selecting a non autologous antigen presenting and/or immunomodulatory system having a significant difference in antigen presentation and/or immunomodulatory capacity for said host subject as compared to autologous antigen presenting cells from said host subject.

The selected system is preferably an allogeneic APC (HP-APC) having high antigen presentation capacity for said subject.

The term "significant difference" indicated in relation with the antigen presentation and/or immunomodulatory capacity of non-autologous systems as well as the term "high performance" or the expression "highly efficient" associated with selected antigen presenting cells indicate, within the context of this invention, that said systems / cells are able to cause a measurable increase or reduction in the subject white blood cell response, i.e., an increase or a reduction in the subject white blood cell response greater than autologous APCs from the subject to be treated. The response amplification level is preferably at least 5 times greater than autologous APCs from the subject, preferably at least 10 times, 20 times, 50 times or, even more preferably, at least 100 times. In a particularly advantageous embodiment, the response amplification level is logarithmic, e.g., at least 10 times, 100 times, 1000 times, 10 000 times, 100 000 times greater than autologous APCs from the subject. The present invention proposes for the first time to select non autologous systems, such as allogeneic APCs, for their antigen presentation and/or immunomodulatory capacity, said systems being intended for vaccination of a given subject and allowing a response amplification level, as described above, without comparison with the response likely to be obtained in said subject with its autologous APCs.

The antigen presentation capacity may be assessed under various conditions. In this respect, in a preferred embodiment, non autologous antigen presenting and/or immunomodulatory systems as defined above are selected having a high capacity to modulate, i.e., stimulate or inhibit, an immune response (specific or non specific), i.e., a high capacity to modulate a particular type of white blood cells such as T cells [in particular T lymphocytes ("T helper"), regulatory T cells, T cytotoxic lymphocytes, etc.], NK cells, K cells, B cells, NKT cells, etc., from the host subject, in particular cells having high capacity to stimulate a CTL response from the subject or cells having high capacity to stimulate a TH1 or TH2 response from the host subject.

In a preferred embodiment, a HP-APC of this invention is a HP-DC having the ability to increase the antigen-specific CTL response of a subject at least 5 times greater than autologous APCs from the host subject.

In another preferred embodiment, a HP-APC of this invention is a HP-DC having the ability to increase the regulatory T cell response of a subject at least 5 times greater than autologous APCs from the host subject.

In a further particular embodiment, a HP-APC of this invention is a HP-DC having the ability to increase the NK cell effector function subject at least 5 times greater than autologous APCs from the host subject.

In a further particular embodiment, a HP-APC of this invention is a HP-DC having the ability to induce a polarized TH1 response in a subject as compared to autologous APCs from the host subject.

In a further particular embodiment, a HP-APC of this invention is a HP-DC having the ability to induce a polarized TH2 response in a subject as compared to autologous APCs from the host subject.

The antigen presentation and/or immunomodulatory capacity of the tested non autologous systems may be assessed by a number of techniques, including biological and/or phenotypic assays or analyses, as will be disclosed below. Furthermore, in a preferred embodiment, the method comprises selecting not only one but at least two, three, five or more distinct non autologous antigen presenting and/or immunomodulating systems (preferably donor APCs), which cause the higher increase or reduction in the subject white blood cell response. This allows the definition of a subsequent treatment protocol using sequentially distinct systems (preferably HP-APCs).

Biological Assays

In a first particular embodiment, the non autologous systems (preferably allogeneic HP-APCs) are produced or selected using at least one biological assay. Typically, the biological assay comprises a contacting step between a non autologous system according to the invention, and a biological sample from the subject, e.g., tissues or cells, preferably white blood cells; and determining the response of said cells. The contacting step is generally performed in the presence of an antigen against which a response is sought.

A particular embodiment of the present invention thus relates to a method for selecting non autologous antigen presenting and/or immunomodulatory systems, preferably high performance allogeneic antigen presenting cells (HP-APCs), wherein the antigen presentation and/or immunomodulatory capacity of the systems is assessed by a biological assay comprising:

(a) contacting in vitro at least one, preferably two non-autologous antigen presenting and/or immunomodulatory system(s) with white blood cells, preferably T cells, from the host subject, preferably in the presence of a selected antigen, and

(b) selecting non autologous antigen presenting and/or immunomodulatory systems (preferably high performance allogeneic APCs) that cause an increase or a reduction in the subject white blood cell response at least 5 times greater than autologous APCs from the subject, preferably at least 10 times, 20 times, 50 times or, even more preferably, at least 100 times, 1000, 10 000 or 100 000 times.

The method described above preferably comprises the selection of at least two non autologous antigen presenting and/or immunomodulatory systems causing the higher increase or reduction in the host subject T cell response.

The selected antigen is preferably selected, in the above described methods, for example from a tumor-associated antigen, a viral antigen, a bacterial antigen, a parasitic antigen, a mycologic antigen, a prion antigen, an allergen, an alloantigen and an auto-antigen.

The white blood cells may include, alone or in combination(s), various cell types such as T lymphocytes, NK cells, B cells or NKT cells. The antigen presentation capacity is preferably assessed with respect to T lymphocytes from a subject.

The white blood cells can be obtained from a sample of whole blood of the subject using any technique known per se in the art, such as an apheresis, an affinity separation (e.g., using monoclonal antibodies directed against the targeted cell surface antigens), etc. In addition (or alternatively), purification of white blood cells may also be performed by countercurrent centrifugal elutriation or affinity separation.

In a more preferred embodiment, the white blood cells are selected from T lymphocytes, NK cells, B cells or NKT cells, from the subject to be treated, even more preferably T cells (such as for instance helper T cells, CTL, Treg, etc.).

In a particular embodiment, the method according to the invention comprises the selection of non autologous antigen presenting and/or immunomodulatory systems, in particular of HP-APCs, that modulate a T cell response, i.e., that increase or decrease a T cell response.

Biological methods that are suitable for measuring an immune function include: methods based on counting the number of white blood cells or different subsets thereof, methods based on measuring the proliferation of said cells, methods based on measurement of the clonal expansion of a specific sub-population, methods based on measurement of antibody secretion, methods based on measurement of cytotoxic activity or secretion of cytokines, methods based on measurement of a TH1 or TH2 response, methods based on measurement of target cell lysis and methods used in vivo, such as skin tests and adoptive transfer. Some of these methods are described in detail in the literature (see for example Groeneveld et al., Journal of the International Federation of Clinical Chemistry, 6: 84-94; 1994; Clough and Roth, JAVMA 206:1208-1216, 1995; Constantin CM Biol Res Nurs. 2002 Oct;4(2):115-27, Jerome KR, Apoptosis. 2003 Dec;8(6):563-71, Moretta A Immunol Today. 2000 May;21(5):228-34., Campbell JD. Methods. 2003 Oct;31 (2):150-9. etc.).

In a preferred embodiment, systems are selected that have a high capacity to modulate, i.e., stimulate or inhibit, a particular type of white blood cells such as T cells [in particular T lymphocytes ("T helper"), regulatory T cells, T cytotoxic lymphocytes, etc.], NK cells, K cells, B cells, NKT cells, etc., from the host subject, in particular systems having high capacity to stimulate a CTL response from the host subject or systems having high capacity to stimulate a TH1 or TH2 response from the host subject, etc.

Effector function that can be used as parameters to evaluate the activity of non autologous systems such as allogeneic HP-APCs include CTL or cytotoxic activities or functions, immunosuppressive activities or functions, cellular activation activities or functions, etc.

A first method that can be used to detect and/or measure an immune response comprises determining the number of white blood cells or subsets thereof upon contacting the APC and white blood cells from the subject, in the presence of the antigen. A variety of techniques have been described in the literature to measure this proliferative response, including immunofluorescence microscopy, immunocytochemistry, enzyme immunoassay, and flow cytometry. Flow cytometry, in particular, is widely used in clinical laboratory settings.

Leukocyte proliferation assays are based on division of responding cells and are typically performed using radioactive isotopes. This technique evaluates the division of a small population of cells, require tissue culture and usually take 3-10 days (Egner W, Adv Exp Med Biol. 1993;329:263-8., Coronel A Br J Haematol. 2001 Sep;114(3): 671-80.). Counting cells and quantification of cell proliferation can also be performed using traditional reagents in particular the Hoechst nucleic acid stains and probes for 5-bromo-2'-deoxyuridine (BrdU) incorporation during cell division. Incorporated BrdU into newly synthesized DNA permits indirect detection of rapidly proliferating cells thereby facilitating the identification of cells that have progressed through the S-phase of the cell cycle during the BrdU labeling period (Kubbies M, J Cell Physiol. 1985 Nov;125(2):229-34.). 5-(and 6-) carboxyfluorescein diacetate succinimidyl ester (CFSE) is used for generation analysis of cells. CFSE spontaneously and irreversibly couples to both intracellular and cell-surface protein. When cells divide, CFSE labelling is distributed equally between the daughter cells, which are therefore half as fluorescent as the parents. As a result, each successive generation in a population of proliferating cells is marked by a halving of cellular fluorescence intensity that is readily detected by a flow cytometer (Putz T, J Clin Immunol. 2004 Nov; 24(6):653-63.).

Cytotoxic T-lymphocytes (CTLs) are believed to be the major host defense mechanism in response to a variety of viral infections and neoplastic or cancerous growth. These cells eliminate infected or transformed cells by recognizing antigen fragments in association with various molecules (termed class I MHC molecules) on the infected or transformed cells. In a preferred embodiment, the method comprises selecting non autologous antigen presenting and/or immunomodulatory systems, in particular allogeneic HP-APCs, that modulate, preferably stimulate a CTL response greater than autologous APC from the subject.

Significant antigen-specific cytotoxic T-lymphocyte responses to a desired antigen may be obtained by contacting the subject's cytotoxic T-lymphocytes to non autologous systems selected or prepared with a method according to the invention or obtained from a bank according to the invention, in particular allogeneic HP-APC, sensitized (transfected, pulsed, etc.) with said particular allogeneic antigen, or in the presence of said selected allogeneic antigen, and then measured using a method as described above.

Enhancing the function of regulatory T-cells in an organism may further be desired to limit the immune T-cell response in those circumstances where such a response is undesirable, such as when a subject suffers from autoimmune disease. Regulatory T cells (Treg) limit autoimmunity but can also attenuate the magnitude of anti-pathogen and anti-tumor immunity. Inhibiting the function of regulatory T-cells in an organism may be used to enhance the immune T-cell response in those circumstances where such a response is desirable, such as in a patient suffering from cancer, chronic infection, or a bone marrow transplant recipient. Therefore, another particular object of the present invention relates to a method as described above comprising the selection or preparation of non autologous systems, in particular allogeneic HP-APC, having high capacity to modulate, i.e., stimulate or inhibit, regulatory T cells from the subject to be treated.

The function of a regulatory T-cell may be, for example, enhanced by enhancing or increasing CD223 activity, or by increasing the number of CD223+ cells in a T-cell population. Conversely, the function of a regulatory T-cell may be inhibited by inhibiting CD223 activity or by decreasing the number of CD223+ cells in a T-cell population. A particular method of the invention may thus comprise the selection of non autologous systems having high capacity to modulate, i.e., stimulate or inhibit, CD223+ cells from the subject to be treated.

Differentiation and expansion of regulatory T cells may also be detected by enhanced or increased CD152 (Boden E et al 2003), GTIR (Mottonen M et al 2005), CD150 (Browning MB et al, 2004) or Fox-P3 (Zelenay S et al, 2005) expression, or by increasing the number of CD152, or GTIR or CD150, or Fox-P3 cells in a T-cell population. Conversely, the function of a regulatory T-cell may be inhibited by inhibiting CD152, GTIR, CD150, or Fox-P3 activity or by decreasing the number of CD152, GTIR, CD150 or Fox-P3 positive cells in a T-cell population. A particular method of the invention may thus comprise the selection of non autologous systems having high capacity to modulate, i.e., stimulate or inhibit, CD152, GTIR, CD150 or Fox-P3 cells from the subject to be treated.

In the present invention, non autologous antigen presenting and/or immunomodulatory systems, in particular allogeneic HP-APC, may also be selected for their high capacity to modulate, preferably induce either a TH1, TH2, TH3 or Tr1-type immune response, preferably a TH1 or TH2-type. An unbalance in Th1/Th2 responses was observed in a variety of clinical situations, for example such as atopic allergy (mediated by IgE), and particularly asthma, allergic rhinitis, and also graft rejects, diseases of the graft against the host, leishmanioses, leprosy, tuberculosis and chronic inflammatory diseases such as Crohn's disease, reactive arthritis, insulino-dependent diabetes.

Appropriate polarisation of either of the responses is crucial to achieve efficient elimination of a particular pathogen. This polarisation requires differentiation of naive CD4+ T cells into Th1/Th2 effector cells. This step is a major element in the decision about the type of immune response (Mossmann et al., 1986; Mossmann et al., 1989; Romagnani, 1999). The aptitude of the different subtypes of T cells to direct immune effector responses is due to an exclusive combination of cytokines expressed partly in a subtype of particular Th cells. Thus, in human and also in the mouse, Th1 cells preferably produce interleukin 2 and interferon gamma; these Th1 cells cause a retarded hypersensitivity type response, while Th2 cells secrete interleukin 4, interleukin 5, interleukin 10 and induce activation of B cells and the production of antibodies.

Such a qualitative response may be addressed for instance by measuring cytokine secretion, for example by measuring IFNy or IL-2 (Th1) and IL4, IL5, IL10 or activation of B cells (Th2) responses, and/or by quantifying IgG2a (Th1) and IgG1 (Th2) antibody responses or by measuring chemokine secretion, for example by measuring MIP-1a, MIP-1b or RANTES secretion.

Natural Killer cells (NK cells) are a population of lymphocytes which represent a very early line of defense against viruses and tumour cells. NK cells can be characterized by the presence of CD56 and CD16 markers and by the absence of the CD3 marker. NK cells are involved in non specific anti-tumoral immunity of antigens, to prevent the establishment of primitive or metastatic tumours in the immunocompetent or immunosuppressed host. In particular, the role of NK cells in anti-tumoral immunosurveillance (primitive tumor or metastases) has been suggested. NK cells appear, in particular, to play a key role against tumor cells or negative class I MHC cell variants. It may thus be desirable; as proposed by the present invention, to select non autologous systems, in particular allogeneic HP-APCs, having high capacity to modulate, preferably stimulate, NK cells from the subject.

As NKT cells can influence immunological responses to antigen in a subject by modulating DC or B cells function for example, it may further be desirable, as proposed by the present invention, to select non autologous systems, in particular allogeneic HP-APCs having high capacity to modulate, stimulate or deplete, NKT cells from a subject. The immune response may be a CD4+ and/or a CD8+ T cell response, an antibody response, a modulation of the Th1/Th2 balance toward anti-inflammatory cytokine producing cells, etc.

NKT cells are T cells with an invariant TCR and intermediate level NK1 surface expression. Although the ligand for such invariant TCR cells has not been defined, it is possible that NKT cells respond to a common glycolipid presented by CDld molecules that is induced as an innate response to cellular distress. It is believed that use of TLR activators in conjunction with non autologous systems, in particular allogeneic HP-APCs, activating NKT cells provide a synergistic adjuvant effect.

Another particular embodiment relates to the selection of non autologous systems, in particular allogeneic HP-APCs, having high capacity to modulate, preferably stimulate, B cells from the subject to be treated.

The immune response mediated by B lymphocytes may be assessed by the detection and/or measure of effective antibody responses. Said antibody may of course be directed against a desired antigen.

As explained above, It may thus also be desirable, as proposed by the present invention, to select non autologous systems, in particular allogeneic HP-APCs, having high capacity to modulate cytokine secretion (using a cross-priming method for example) or antibody production.

A preferred method according to the invention is a method as described previously wherein the immune response is selected from T cell expansion, antigen specific cytotoxic T cell response, TH1 response, TH2 response, induction of cytokine secretion, induction of regulatory T cells, induction of NK cells, NKT cells or B cells.

In a particular embodiment, the invention also relates to a method, as previously described, wherein target cells are added to non autologous systems, in particular allogeneic HP-APCs, and white blood cells, preferably T lymphocytes, after the contacting step (a) and wherein the immune response is assessed by measuring an effector function, preferably by measuring target cells lysis.

The biological assay may also comprise the identification / selection of the couple non autologous system (in particular allogeneic HP-APC) / white blood cells from a donor allowing the higher increase or reduction in the donor immune response, in order to identify, in the host subject to be treated, the type of white blood cell which will be used to assess or influence the subject's immune response. Such identification / selection may be realized as described previously.

Assay making it possible to verify that non autologous systems, in particular allogeneic HP-APCs, possess the capacity, when they are inoculated, to reverse a disease, e.g., to cause established solid tumors or infected tissue to recover, regress or disappear, may also be carried out as a biological assay according to the invention.

Phenotypic Analysis

The selection of the non autologous systems, in particular allogeneic HP-APCs, can also be carried out by phenotypic analysis. Such analysis may be performed either alone, or in combination with a biological assay as described above. For instance, the HLA or marker phenotype of the cells may be selected first by phenotypic analysis, to include only a limited number of cells in a biological assay as discussed above. In a preferred embodiment, the non autologous systems, in particular allogeneic HP-APCs, are selected based solely on phenotypic analysis, as discussed in the present section. In this specific embodiment, there is no need to perform biological testing or co-culture of the subject white blood cells with non autologous systems from a bank according to the invention, except for validation purposes.

In the context of the present invention, a phenotypic analysis comprises the determination of the presence of particular characteristics within the APCs and the subject's white blood cells, that define a high antigen presentation and/or immunomodulatory capacity. Such phenotypic analysis is preferably an HLA allele analysis (e.g. the identification of HP pairs of alleles) or a marker analysis. A non autologous system, in particular an allogeneic HP-APC, may express a phenotypic marker of interest as described previously or a combination of such phenotypic markers, preferably correlated to a desired function (e.g. white blood cell amplification, etc.) or be of a particular HLA phenotype.

The phenotypic analysis typically comprises a fist step of determining the phenotype of the subject's white blood cells, for instance the HLA haplotype, the expression of particular adhesion or co-stimulatory or accessory molecules at the surface of said cells. The bank can then be scanned or analysed to identify systems having appropriate phenotype [e.g. APC expressing particular markers adhesion or chemo-attraction molecule(s) or having a genetic profile or specific intracellular transport / specific localization ("trafficking/homing") systems or coactivation molecule(s)] to define a non autologous system, in particular an allogeneic HP-APC, with respect to said subject.

Examples of phenotyping molecules may be any B7:CD28 family members, the DEC-205 receptor, the DC-Lamp (CD208), any Toll-like receptors (TLRs) or the DC-SIGN (dendritic cell-specific ICAM-grabbing non-integrin).

B7:CD28 family members regulates T cell activation and tolerance. B7-1/B7-2:CD28 interactions not only promote initial T cell activation but also regulate self-tolerance by supporting CD4(+)CD25(+) T regulatory cell homeostasis. The five B7 family members, ICOS ligand, PD-L1 (B7-H1). PD-L2 (B7-DC), B7-H3, and B7-H4 (B7x/B7-S1) are expressed on professional antigen-presenting cells, providing new means for regulating T cell activation and tolerance in peripheral tissues (Greenwald RJ et al, 2005).

DEC-205 receptor is an abundant endocytic putative antigen uptake receptor present on APC in lymphoid tissues. DEC-205 belongs to a family of transmembrane C-type lectins. This molecule is known to be one of the most authentic markers for the lineage of dendritic cells (Bonifaz LC et al, 2004). DC-Lamp (CD208) is a lysosome-associated membrane glycoprotein (Salaun B et al, 2003).

Toll-like receptors (TLRs) are type I transmembrane proteins involved in innate immunity which recognize microbial conserved structures. TLR3, TLR7 and TLR9 may play an important role in detecting and combating viral infections (Michelsen KS et al, 2003).

DC-SIGN (dendritic cell-specific ICAM-grabbing non-integrin, where ICAM is intercellular adhesion molecule) is a recently described mannose-specific C-type lectin expressed by dendritic cells. DC-SIGN, which is expressed by dendritic cells, binds to ICAM-3 on T-lymphocytes, therefore playing an important role in the activation of T-lymphocytes (Soilleux EJ, 2003).

As discussed above, the method comprises selecting either one or several (e.g. two, three, five or more) distinct non autologous systems, in particular allogeneic APCs, which cause the higher increase or reduction in the subject white blood cell response. The capacity of these cells may be further validated in any appropriate system, such as any in vivo assay (in non-human animal) or addition biological assays. The selected non autologous systems, in particular allogeneic cells, may then be identified, expanded, processed or otherwise treated for subsequent use for treating the host subject by allogeneic vaccination.

**Pharmaceutical Compositions**

Another object of the invention relates to a pharmaceutical composition, e.g., a vaccine, for modulating an immune response in a host subject, wherein said composition comprises a non autologous antigen presenting and/or immunomodulatory system, for example an allogeneic APC or a membrane material derived therefrom, and a pharmaceutically acceptable excipient or diluent. Preferably, said system causes in vitro an increase or a reduction in the host subject T cell response at least 5 times greater than autologous APCs from said subject, preferably at least 10 times, 20 times, 50 times or, even more preferably, at least 100 times, 1000 times, 10000 times or 100000 times.

The non autologous antigen presenting and/or immunomodulatory system (for example an allogeneic APC or a membrane material derived therefrom) of the composition described above even more preferably shares at least one MHC haplotype with said subject. They further preferably comprise at least one selected antigen.

A particularly preferred allogeneic cellular vaccine, as used herein, is a preparation which contains an allogeneic HP-APC or a membrane material derived therefrom which expresses one or more specific antigen and has a high ability to induce antigen-specific cytotoxic T lymphocytes in a given patient.

A further embodiment of the present invention relates to a pharmaceutical composition for modulating an immune response in a host subject, wherein said composition comprises an immune cell activated by a non autologous antigen presenting and/or immunomodulatory system and a pharmaceutically acceptable excipient or diluent.

The immune cell may be selected for example from T cell, NK cell, NKT cell, B cell, etc. To be used in the preparation of a composition according to the invention, the immune cell has to be activated by a non autologous antigen presenting and/or immunomodulatory system preferably selected by a method according to the invention and preferably loaded with at least one desired antigen. Activated immune cells are preferably further amplified before being introduced in the pharmaceutical composition.

The non autologous antigen presenting and/or immunomodulatory system (for example an allogeneic high performance APCs) or the activated immune cell may be suspended in any pharmaceutically acceptable carrier, excipient or diluent. The pharmaceutically compatible or physiologically acceptable carrier, excipient or diluent may be selected from neutral to slightly acidic, isotonic, buffered saline (including phosphates, chloride, etc.) solutions or suspensions and more preferably from sucrose, trehalose, surfactants, proteins and amino acids. The pharmaceutically compatible carrier, excipient or diluent is preferably contained in an appropriate buffer to form an isotonic solution.

The pharmaceutical composition may comprise additional active ingredients or adjuvants. The adjuvant may be selected from any substance, mixture, solute or composition facilitating or increasing the immunogenicity of an antigen and able to induce a Th1-type immune response, such as a hematopoietic cell growth factor, a cytokine, CpG, QS21, ISCOM and monophosphoryl lipid A for example. Such adjuvants are particularly suited to produce and/or amplify a specific immune response against antigen(s) in mammalian subjects, particularly in humans. The adjuvant may be conditioned and administered separately or sequentially from the HP-APCs containing composition and/or at a distinct site of injection.

Any suitable therapeutic agents can be coordinated with the treatments of the present invention. For example, any suitable anti-tumor composition or treatment can be coordinated with the treatments of the present invention targeted to cancer cells. Similarly, if treating patients with infections, other anti-infectious agents can be coordinated with the treatment of the present invention targeted to infected cells. Such agents may be for example small molecule drugs, vaccines, antibodies, etc.

Actual dosage levels of active ingredients in the compositions of the present invention may be adapted so as to obtain an amount of active ingredient that is effective to obtain a desired immune response for a particular composition and method of administration. The selected dosage level therefore depends upon the route of administration, the desired duration of treatment and other factors.

It should be understood, however, that the specific dose level for any particular patient will depend upon a variety of factors including the body weight, general health, sex, diet, time and route of administration, rates of absorption and excretion, combination with other drugs and the severity of the particular disease being treated.

Preferred administration routes are parenteral routes. The parenteral route is preferably an intra-tumoral, more preferably an intra-venous, an intra-dermic, a cutaneous or sub-cutaneous administration. It includes also intra-arterial, intraperitoneal, intra-tracheal or intra-muscular injections. It should be understood, however, that any other suitable administration route may be contemplated depending upon the health status and the reactivity of the patient.

The preferred subcutaneous injection volume, for example, is between 0.01 ml and 1ml or 5 ml, preferably between 0.05 ml and 0.5 ml, containing 1 to $50 \times 10^6$ allogeneic cells; however, as explained above, other injection volumes and cell concentrations may be used. One skilled in the art can easily make these modifications to injection volume and cell concentration as required for the particular allogeneic cell type and the particular mammal being immunized.

The pharmaceutical compositions may conveniently be presented in unit dosage form and may be prepared by any of the methods well-known in the art of pharmacy. All methods include the step of bringing the active agent into association with a carrier as described above which constitutes one or more accessory ingredients. In general, the compositions are prepared by uniformly and intimately bringing the active agent into association with a liquid carrier, a finely divided solid carrier, or both, and then, if necessary, shaping the product.

Compositions suitable for oral administration may be presented as discrete units, such as capsules, tablets, lozenges, each containing a predetermined amount of the active agent. Other compositions include suspensions in aqueous liquids or non-aqueous liquids such as a syrup, elixir or an emulsion.

Other delivery systems can include time-release, delayed release or sustained release delivery systems. Such systems can avoid, when required, repeated administrations of the active agent, increasing convenience to the subject and the physician. Many types of release delivery systems are available and known to those of ordinary skill in the art. They include polymer base systems such as poly (lactide-glycolide), copolyoxalates, polycaprolactones, polyesteramides, polyorthoesters, polyhydroxybutyric acid, and polyanhydrides. Delivery systems also include non-polymer systems that are: lipids including sterols such as cholesterol, cholesterol esters and fatty acids or neutral fats such as mono- di- and tri-glycerides; hydrogel release systems; sylastic systems; peptide based systems; wax coatings; compressed tablets using conventional binders and excipients; partially fused implants; and the like. In addition, pump-based hardware delivery systems can be used, some of which are adapted for implantation.

Use of a long-term sustained release implant may be particularly suitable for treatment of chronic conditions. Long-term release, are used herein, means that the implant is constructed and arranged to deliver therapeutic levels of the active ingredient for at least 30 days, and preferably 60 days. Long-term sustained release implants are well-known to those of ordinary skill in the art and include some of the release systems described above.

Another embodiment of the present invention relates to the use of a non autologous antigen presenting and/or immunomodulatory system, for example an allogeneic (preferably human) high performance APC or a membrane material derived therefrom, for the preparation of a pharmaceutical composition as described above for the modulation of an immune response in a subject, wherein said system causes in vitro an increase or a reduction in the subject white blood cell response, preferably T cell response, at least 5 times greater than autologous APCs from the subject, preferably at least 10 times, 20 times, 50 times or, even more preferably, at least 100 times, 1000 times, 10 000 times or 100 000 times, and wherein said system preferably shares at least one MHC haplotype with said subject.

The non autologous antigen presenting and/or immunomodulatory system (such as an allogeneic high performance APC or a membrane material derived therefrom) may be obtained by a method or from a bank according to the invention. As indicated before, the system may preferably expressed an antigen. Such a system (for example HP-APCs) may be obtained from a unique donor or from at least two donors and are preferably pulsed with one or several antigens.

A further embodiment of the present invention relates to the use of an immune cell activated by a non autologous antigen presenting and/or immunomodulatory system as mentioned previously, preferably selected by a method or from a bank according to the invention, for the preparation of a pharmaceutical composition for the modulation of an immune response in a subject.

Another embodiment of the present invention relates to a method for preparing a composition for modulating an immune response in a subject, the method comprising:

(a) providing a library of non autologous antigen presenting and/or immunomodulatory systems, preferably mammalian cells, as previously described ;
(b) assessing the antigen presentation and/or immunomodulatory capacity of systems from the library towards white blood cells from the subject as previously described,
(c) selecting systems from said library having a significant difference in antigen presentation and/or immunomodulatory capacity for said host as compared to autologous antigen presenting cells from said host subject, as previously described, and
(d) preparing a composition according to the invention using systems selected in step (c).

A pharmaceutical composition according to the invention may be useful for treating organisms suffering from conditions resulting in a low T-cell population. Such conditions include diseases resulting from immunodeficiency such as AIDS, as well as disorders involving unwanted cellular invasion or growth, such as invasion of the body by foreign microorganisms (bacteria or viruses) or tumor growth or cancer.
The invention is thus particularly suited to produce a preventive or curative immune response in subjects, such as immunodeficient patients, cancer patients, patients undergoing grafts, patients infected with a virus or a parasite, elderly patients or any patients having low CD4 count etc.

Another embodiment of the present invention therefore relates to a method (vaccination) for modulating an immune response to an antigen in a host subject, preferably a human, the method comprising administering to the subject a composition according to the invention comprising a non autologous antigen presenting and/or immunomodulatory system (for example non autologous or allogeneic APCs, preferably allogeneic high performance APCs, or a membrane material derived therefrom), preferably obtained by a method or from a bank according to the invention, wherein said system causes in vitro an increase or a reduction in the subject white blood cell (preferably T cells) response at least 5 times greater than autologous APCs from the subject, preferably at least 10 times, 20 times, 50 times or, even more preferably, at least 100 times, 1000, 10 000 or 100 000 times, and wherein said system preferably shares at least one MHC haplotype with said subject.

Vaccination, as used herein, refers to the step of administering the vaccine, preferably an allogeneic cellular vaccine, to a mammal to induce such an immune response.

Preferably, the modulated immune response is selected from T cell expansion, peptide specific cytotoxic T cell response, TH1 response, TH2 response, induction of cytokine secretion, induction of regulatory T cells, induction of NK cells, NKT cells and B cells.

The method contemplates both single and multiple immunizations.
Treatment with multiple agents according to the invention need not be done using a mixture of agents but may be done using separate pharmaceutical preparations. The preparations need not be delivered at the same exact time, but may be coordinated to be delivered to a patient during the same period of treatment, i. e., within a week or a month or each other. Identical or different kind of non autologous antigen presenting and/or immunomodulatory systems such as non autologous cells, preferably allogeneic cells, even more preferably high performance APCs, may indeed be administered simultaneously or sequentially.

In a particular embodiment, the present invention relates to a method for modulating an immune response to an antigen in a host subject, wherein non autologous antigen presenting and/or immunomodulatory systems such as non autologous cells, preferably allogeneic cells, even more preferably high performance APCs, may be from a unique donor and may be pulsed with one or several identical or distinct antigens. Said cells may also be from distinct donors, for example from at least two donors. Said cells being from distinct donors and/or having distinct antigens may be administered simultaneously or sequentially.
Preferably, methods according to the invention are for stimulating an antigen specific immune response.

In this respect, the non autologous antigen presenting and/or immunomodulatory system, in particular and for example the HP-APC, may be modified (e.g., genetically or loaded) with an antigen against which an immune response is sought. This antigen may be any antigen (i. e., a substance that when introduced into the body stimulates the production of an immune response, e. g. an antibody response), including any synthetic or natural antigen. The antigen may be an alloantigen. The antigen can be of any type including, for example, nucleic acids (e.g., DNA, RNA such as unfractionated tumor-derived antigens in the form of RNA), hormones, allergens, entire proteins, peptides, particularly peptides that are presented to the immune system through MHC class I or MHC class II molecules, any epitope-containing fragment, recombinant proteins, a killed, inactivated or attenuated pathogen product, cellular material (e. g. , live or inactivated allogeneic cancer cells), particulate matter such as, but not limited to, cell debris, apoptotic cells, lipid aggregates such as liposomes, membranous vehicles, microspheres, heat aggregated proteins, virosomes, virus-like particles and whole organisms including, for example, parasite, bacteria, mycobacteria, viruses, fungi, protozoa, fungi, a portion thereof and a combination thereof.

Specific examples of antigens include antigens derived from HIV, Varicella Zoster virus, Influenza virus, Epstein Barr virus, type I or 2 Herpes Simplex virus, human cytomegalovirus, Dengue virus, Hepatite A, B, C or E virus, Syncytium respiratory virus, human papilloma virus, plasmodium falciparum plasmodium malariae plasmodium vivax plasmodium ovale, mycobacterium tuberculosis, Toxoplasma and Chlamydia, any tumor-specific antigen expressed by a tumor cell, such as cells from melanomas, squamous cell carcinomas, adenocarcinomas, hepatocellular carcinomas, renal cell carcinomas, sarcomas, myosarcomas, leukemias, lymphomas, central nervous system tumors, etc.

In a particular embodiment, the invention also relates to a method for modulating an immune response to an antigen in a host subject, the method comprising administering to the subject a composition comprising an immune cell activated (as described previously) by a non autologous antigen presenting and/or immunomodulatory system preferably selected by a method according to the invention.

**Method for optimizing an antigen preparation**

Another object of the invention relates to a method for optimizing an antigen preparation by comparing an immune response to said antigen preparation and to one or several variant antigen preparations thereof, wherein the immune response is determined by contacting said antigen preparations with at least one non autologous antigen presenting and/or immunomodulatory system, preferably with at least two nonon autologous antigen presenting and/or immunomodulatory systems having a significant difference in antigen presentation capacity towards white blood cells from a host subject as described above, and selecting variant antigen preparation(s) causing an optimized immune response.

Non autologous antigen presenting and/or immunomodulatory systems are preferably antigen presenting cells, preferably selected by a method according to the invention or from a bank according to the invention.

In a preferred embodiment, antigen preparations may also be contacted with T cells from a host subject, the antigen specific cytotoxic T cell response being preferably determined.

In a particularly preferred method, antigen preparations are contacted with T cells from a host subject and at least two antigen presenting cells, preferably obtained from a method or from a bank according to the invention, having a significant difference in antigen presentation capacity towards immune cells or white blood cells, preferably T cells, from the host subject, the selected variant antigen preparation(s) causing an optimized T cell response.

The antigen preparation or a variant thereof may comprise a wild type antigen, a mutated antigen, a glycosylated antigen, a part thereof, a composition comprising an antigen and any other compound as described above in relation with the pharmaceutical composition according to the invention, such as a compound exhibiting a different biological activity, an adjuvant, any impurity, etc., or a mixture thereof.

### Method for selecting a host responder subject

A further aspect of this invention is a method for selecting a host subject that responds to an immunotherapeutic treatment, comprising a step of contacting immune cells, preferably T cells, from said subject, in the presence of said immunotherapeutic treatment, with at least one non autologous antigen presenting and/or immunomodulatory system, preferably at least two antigen presenting and/or immunomodulatory systems having a significant difference in antigen presentation capacity towards immune or white blood cells, preferably T cells, from the host subject as described above, and assessing the response of said immune cells, said response being indicative of a responder subject.

Non autologous antigen presenting and/or immunomodulatory systems are preferably antigen presenting cells, preferably selected by a method according to the invention or from a bank according to the invention.

When immune cells are T cells, the immune response assessed is preferably an antigen cytotoxic T cell response.

A preferred immunotherapeutic treatment is a method (vaccination) according to the invention for modulating an immune response preferably using a pharmaceutical composition according to the invention.

### Kits

In accordance with the methods of the present invention, kits for evaluating the immune responses are envisioned.

If a known active substance or composition and the vaccine according to the invention are to be administered in a time-separated fashion, they may be supplied together in a kit. Within the kit, the components may be separately packaged or contained. Other components such as excipients, carriers, other immune modulators or adjuvants, instructions for administration of the known active substance or composition and the vaccine, and injection devices can be supplied in the kit as well. Instructions can be in a written, video, or audio form, can be contained on paper, an electronic medium, or even as a reference to another source, such as a website or reference manual.

In particular, the invention includes a kit containing antigens or mitogens or other inducing agents either in liquid or lyophilized form, paramagnetic beads coupled with an antibody for isolation of the predetermined subset of cells, cell culture media for dilution of samples, wash buffer for washing complexes, and associated reagents for performing the assay.

Other aspects and advantages of the present invention will be described in the following examples, which should be regarded as illustrative and not limiting the scope of the present application.

EXAMPLES

### Example 1 : Selection of HP-APC that augment mart-1 specific CD8 positive T cell number.

A panel of 7 random allogeneic DC were compared to autologous DC for their capability to induce mart-1 specific CD8 positive T cells. In that aim, DC were pulsed in RPMI 1640 supplemented with 2 mM L-glutamine, 5% human pooled AB serum, for 1 h at 37°C with 10 μg/ml of the HLA*A0201-restricted mart-1 peptide (ELAGIGILTV). Then, cells were cultured with purified T lymphocytes at a DC/T cell ratio of 1/3 in round bottom 96-well culture plates. On day 1 and 3, IL-2 (30 IU/ml) was added to the culture. On day 7, cells were harvested and mart-1 CD8 positive T cell expansion was evaluated using tetramer staining. T cells were incubated for 30 min with mart-1 HLA-A*0201 class I tetramer (PE conjugated) and with anti-CD8 conjugated with FITC. After a two step wash, samples were analyzed using an EPICS XL flow-cytometer (Coulter, Villepinte, France) (Fig 1).
*The results shown in figure 1 demonstrate that mart-1 presentation using allogeneic DC induced enhanced tumor*

*specific cell number when compared to autologous DC. These results also allow HP-DC selection since a huge variability (ranging from 1.5 to 38i) is observed when a panel of random allogeneic DC is used as CPA.*

**Example 2 : Selection of HP-APC that increase influenza M1 specific CTL number.**

A panel of 7 random allogeneic DC were compared to autologous DC for their capability to induce influenza M1 specific CTL. DC were pulsed in serum free AIM-V medium supplemented with 1% pyruvate for 3 h at 37°C with 10 $\mu$g/ml of the HLA*A0201-restricted influenza M1 peptide. Then, cells were cultured with purified T lymphocytes at a DC/T cell ratio of 1/10 in 48-well culture plates. On day 3, 10 IU/ml IL-2 were added to the culture. On day 8, effector cells were harvested and viable trypan blue-excluded cells were evaluated for their specific lytic activity in a chromium release assay.

Cytolytic activity of effector cells was measured using a conventional 4-hour $^{51}$Cr release assay. In brief, TAP$^{-/-}$, HLA*A0201 MHC class II-negative T2 cell line were pulsed for 1 hour with 4 $\mu$g/ml influenza M1 peptide (GILGFVFTL). Control HLA*A0201-restricted peptide was the melanoma martT-1 epitope (AAGIGILTV). Non pulsed or pulsed target cells were then labeled with $Na_2{}^{51}CrO_4$ (CIS International, Gif sur Yvette, France), washed and incubated at 37°C for 4 hours with $1.5 \times 10^3$ target cells at effector/target ratios ranging from 100/1 to 1/1 in 200 $\mu$l of RPMI 1640 supplemented with 10% foetal calf serum in round-bottomed 96-well microtitre plates. An excess cold K562 target cells (1/30) was used to inhibit NK-mediated lysis. Then, 100 $\mu$l of supernatant was collected and the specific lysis was evaluated using the formula :

$$(cpm_{\text{experiment}} - cpm_{\text{spontaneous release}}) / (cpm_{\text{maximum release}} - cpm_{\text{spontaneous release}}) \times 100$$

Percentages of specific lysis were plotted against the effector/target ratios. By extrapolation from such dose-response curves, one lytic unit 50 ($LU_{50}$) is arbitrarily defined as the cytotoxic activity required to achieve 50% specific lysis of the labeled target cells under the standard assay conditions described above. LU/culture could be calculated since viable cell recovery per culture had also been determined. The number of LU/culture is considered to provide a relative measure of the number of CTL generated in the cultured cell populations (Fig 2).

*The results shown in figure 2 demonstrate that influenza M1 presentation using allogeneic DC induced enhanced tumor specific CTL number when compared to autologous DC. These results also allow HP-DC selection since a huge variability (ranging from 2 to 10) is observed when a panel of random allogeneic DC is used as CPA.*

**Example 3: Selection of HP-APC that amplify regulatory T cell differentiation.**

To fully exploit the utility of the present invention, various allogeneic DC were assessed for their ability to induce regulatory T cell differentiation.

Lymphocytes from 4 different donors were cocultured with either autologous or a panel of random allogeneic DC for 7 days. At the end of the culture period, cells were enumerated, washed with culture media and stained with mouse monoclonal antibodies to human CD4 (SFC112T4D11), CD25 (B1.49.9) and CTL-A4 (BNI3). Cells were incubated with antibodies for 30 minutes at 4°C. Cells were washed 2 times and analyzed in an EPICS XL flow-cytometer (Coulter, Villepinte, France) (Fig. 3).

*The results shown in figure 3 demonstrate that allogeneic DC induced enhanced regulatory T cell differentiation when compared to autologous DC. These results also allow HP-DC selection since a huge variability (ranging from 3 to 62) is observed when a panel of random allogeneic DC is used as CPA.*

**Example 4 : Selection of HP-APC that enhance NK cell proliferation.**

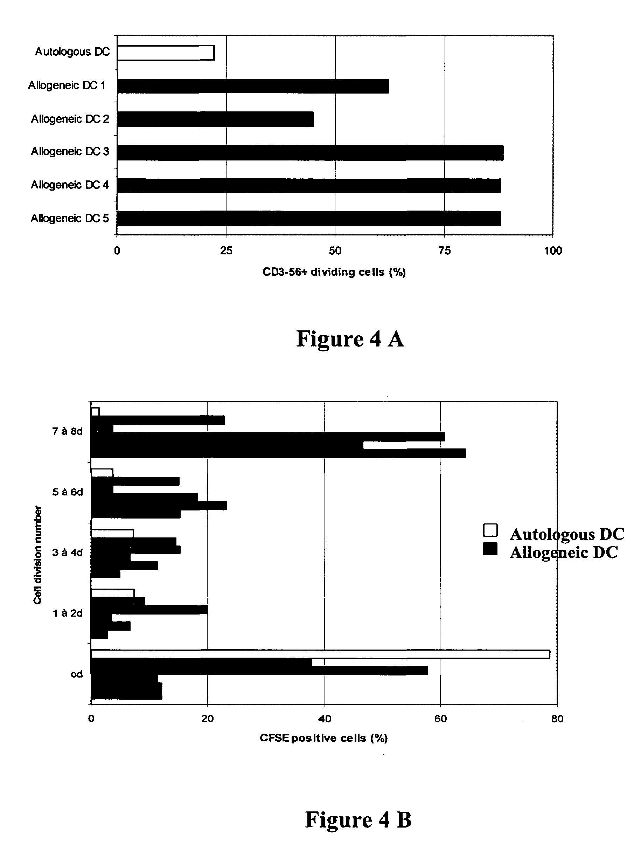

PBL were resuspended at $5 \times 10^6$/ml, without serum and incubated with 5-(and 6-) carboxyfluorescein diacetate succinimidyl ester (CFSE) (Molecular Probes, Eugene, OR) for 10 min at 37°C. Unincorporated dye was immediately quenched by adding an equal volume of FCS. Cells were then extensively washed and cocultured with either autologous DC or a panel of 5 random allogeneic DC. After seven days the CFSE content was monitored on viable cells using an EPICS XL flow-cytometer and the percentage of viable dividing NK cell was determined after staining with PE conjugated anti-CD56, and ECD conjugated anti-CD3 (Beckman coulter, Villepinte, France). Results were both expressed as the percentage of dividing CD3$^-$CD56$^+$ cells (Fig 4 A) and as the CD3$^-$CD56$^+$ cell distribution according to the number of cell division (Fig 4 B).

Fig. 4A reflects a pourcentage of dividing cells and Fig. 4B a cell division number.

*The results shown in figure 4 A and B demonstrate that allogeneic DC induced enhanced CD3-CD56+ cell proliferation when compared to autologous DC. These results allow HP-DC selection.*

### Example 5 : Selection of HP-APC that enhance NK cell activation.

PBL were resuspended at $2 \times 10^6$/ml and cultured in 24-well plates with either autologous DC or a panel of random allogeneic DC in the presence of interleukin 2 (1000 IU/ml) at a DC to effector cell ratio of 1:4. After 7 days, cell were analyzed according to size and granulosity and after 3 color staining using either anti-CD69-FITC, anti-CD56-PE and anti-CD3-ECD (Fig. 5A), or anti-CD25-FITC, anti-CD56-PE and anti-CD3-ECD (Fig. 5B), and using an EPICS XL flow-cytometer (Coulter, Villepinte, France).
*The results shown in figure 5A and B demonstrate that allogeneic DC induced enhanced CD3-CD56+ cell activation when compared to autologous DC. These results also allow HP-DC selection.*

### Example 6 : Selection of HP-APC that enhance NK cell effector function.

Purified PBL were resuspended at $2 \times 10^6$/ml and cultured in 24-well plates with either autologous or a panel of 11 random allogeneic DC in the presence of interleukin 2 (1000 IU/ml) at a DC to effector cell ratio of 1:4. For cytotoxic assays, cells were collected after 7 days of coculture. Viable trypan blue-excluded cells were counted and used as effector cells. Tumoral K562 target cells were then labeled with $Na_2{}^{51}CrO_4$ (CIS International, Gif sur Yvette, France), washed and incubated at 37°C for 4 hours at effector/target ratios ranging from 100/1 to 1/1 in 200 $\mu$l of RPMI 1640 supplemented with 10% foetal calf serum in round-bottomed 96-well microtitre plates. Then 100 $\mu$l of supernatant was collected and the specific lysis was evaluated using the formula :

$$(cpm_{\text{experiment}} - cpm_{\text{spontaneous release}}) / (cpm_{\text{maximum release}} - cpm_{\text{spontaneous release}}) \times 100$$

Percentages of specific lysis were plotted against the effector/target ratios. By extrapolation from such dose-response curves, one lytic unit 50 ($LU_{50}$) is arbitrarily defined as the cytotoxic activity required to achieve 50% specific lysis of the labeled target cells under the standard assay conditions described above. LU/culture could be calculated since viable cell recovery per culture had also been determined. The number of LU/culture is considered to provide a relative measure of the number of NK cell generated in the cultured cell populations (Fig 6).
*The results shown in figure 2 demonstrate that allogeneic DC induced enhanced NK function when compared to autologous DC. These results also allow HP-DC selection since a huge variability (ranging from 1.5 to 6.4) is observed when a panel of random allogeneic DC is used as CPA.*

### Example 7 : Selection of HP-APC based on their migratory capacities.

A panel of 8 random allogeneic DC were compared to autologous DC for their migratory capabilities. The transwell™ system (Costar, Cambridge, UK) was used for migration assays. Bottom compartments of the chamber were filled with 0.6 ml of AIM-V. DC ($4 \times 10^5$) were suspended in 0.2 ml AIM-V and added to the upper compartments. Chambers were incubated for 4 h at 37°C and contents of the lower compartments were collected and counted using the trypan blue exclusion assay (Fig 7). In order to study active migration in response to chemoattractant macrophage inflammatory protein 3 beta 100 ng/ml (R&D systems) were added to the lower compartment of Transwell units.
*The results shown in figure 7 allow HP-DC selection based on their migratory capabilities since a huge variability (ranging from 1 to 9) is observed when a panel of random allogeneic DC is compared.*

### Example 8 : Use of a panel of HP-APC to evaluate donor sensibility to immunotherapy.

A panel of 3 allogeneic DC is used to induce mart-1 specific CD8 positive T cell expansion in order to evaluate healthy donor lymphocyte sensibility to specific immunotherapy. In that aim, DC were pulsed in RPMI 1640 supplemented with 2 mM L-glutamine, 5% human pooled AB serum, for 1 h at 37°C with 10 $\mu$g/ml of the HLA*A0201-restricted Mart-1 peptide (ELAGIGILTV). Then, cells were cultured with T lymphocytes purified from 8 healthy volunteers at a DC/T cell ratio of 1/3 in round bottom 96-well culture plates. On day 1 and 3, IL-2 (30 IU/ml) was added to the culture. On day 7, cells were harvested and Mart1-CD8 positive T cell expansion was evaluated using tetramer staining. T cells were incubated for 30 min with either M1 HLA-A*0201 class I tetramer or HIV-gag HLA-A*0201 class I tetramer (PE conjugated) and with anti-CD8 conjugated with FITC. After a two step wash, samples were analyzed using flow cytometry. A specific clonal expansion score was calculated by adding up the specific mart-1 CD8 positive cell frequency induced by all three DC (Fig 8)

*The results shown in figure 8 demonstrate a huge variability (ranging from 0.05 to 2.4) in the clonal expansion score of 8 distinct PBL suspensions. These results thus allow candidate receiver selection for immunotherapy using a panel of HP allogeneic DC.*

**Example 9 : Selection of HP-APC with high antigen capture capabilities.**

To assess phagocytosis, a suspension of 2 $\mu$m FITC-dextran microbeads (Polysciences, Warrington, PA) was added in the culture media. After a 4 h incubation period at 37°C, cells were recovered and extensively washed with cold Dulbecco's PBS to stop phagocytosis. Then, cells were resuspended in PBS at a final concentration of $5 \times 10^5$ cells/ml and analysed by flow cytometry. in this assay, cell fluorescence intensity depends on the number of beads that have been phagocytised. Therefore, when the number of cells is analysed as a function of the fluorescence intensity, each peak depicted clusters of cells that have phagocytised 1, 2, 3 or more microbeads.

Quantification of endocytic activity was determined by using FITC-dextran internalisation. DC were incubated with 1 mg/ml FITC-dextran (Interchim, Montluçon, France) in RPMI 10% FCS for 30 min at 37°C. Cells were extensively washed with cold PBS containing 1% FCS and 0.01 % NaN3, then analysed on the flow cytometer.

**Claims**

1. A method for selecting a non autologous antigen presenting and/or immunomodulatory system, the method comprising:

   - assessing the antigen presentation and/or immunomodulatory capacity of at least two non autologous antigen presenting and/or immunomodulatory systems towards white blood cells from a host subject, and
   - selecting a non autologous antigen presenting and/or immunomodulatory system having a significant difference in antigen presentation and/or immunomodulatory capacity for said host subject as compared to autologous antigen presenting cells from said host subject.

2. A method according to claim 1, wherein the non autologous antigen presenting and/or immunomodulatory system is selected from a mammalian cell, a recombinant cell, a liposome and a polymer.

3. A method according to claim 1 or 2, wherein the mammalian cell is an antigen presenting mammalian cell.

4. A method according to claim 3, wherein the antigen presenting mammalian cell is selected from dendritic cells, B cells, mast cells, monocytes, or precursors thereof.

5. A method according to claim 3 or 4, wherein the non-autologous antigen presenting and/or immunomodulatory cell is HLA-phenotyped.

6. A method according to anyone of claims 1 to 5, comprising selecting a non autologous antigen presenting and/or immunomodulatory system having high capacity to modulate an immune response from the host subject.

7. A method according to claim 6, comprising selecting a non autologous antigen presenting and/or immunomodulatory system having high capacity to stimulate the specific immune response from the host subject.

8. A method according to claim 6, comprising selecting a non autologous antigen presenting and/or immunomodulatory system having high capacity to stimulate the non specific immune response from the host subject.

9. A method according to claim 6, comprising selecting a non autologous antigen presenting and/or immunomodulatory system having high capacity to stimulate NK cells from the host subject.

10. A method according to claim 6, comprising selecting a non autologous antigen presenting and/or immunomodulatory system having high capacity to stimulate a CTL response from the host subject.

11. A method according to claim 6, comprising selecting a non autologous antigen presenting and/or immunomodulatory system having high capacity to stimulate a TH1 response from the host subject.

12. A method according to claim 6, comprising selecting a non autologous antigen presenting and/or immunomodulatory system having high capacity to stimulate a TH2 response from the host subject.

13. A method according to anyone of claims 1 to 5, comprising selecting a non autologous antigen presenting and/or immunomodulatory system having high capacity to stimulate regulatory T cells from the host subject.

14. A method according to anyone of claims 1 to 4, comprising selecting a non autologous antigen presenting and/or immunomodulatory system having high capacity to stimulate NKT cells or B cells from the host subject.

15. A method according to anyone of claims 1 to 14, wherein the antigen presentation and/or immunostimulatory capacity of the non autologous antigen presenting and/or immunomodulatory system is assessed by a biological assay.

16. A method according to anyone of claims 1 to 14, wherein the antigen presentation and/or immunostimulatory capacity of the non autologous antigen presenting and/or immunomodulatory system is assessed by phenotypic analysis.

17. A method according to claim 16, wherein the phenotypic analysis is an HLA allele analysis.

18. A method according to claim 16, wherein the phenotypic analysis is a marker analysis.

19. A method according to claim 15, wherein the biological assay comprises:

(a) contacting in vitro at least two non autologous antigen presenting and/or immunomodulatory systems with T cells from the host subject, in the presence of a selected antigen, and
(b) selecting non autologous antigen presenting and/or immunomodulatory systems that cause an increase or a reduction in the host subject T cell response at least 5 times greater than autologous APCs from the subject, preferably at least 10 times, 20 times, 50 times or, even more preferably, at least 100 times.

20. A method according to claim 19, comprising selecting at least two non autologous antigen presenting and/or immunomodulatory systems causing the higher increase or reduction in the host subject T cell response.

21. A method according to claim 19 or 20, wherein the antigen is selected from a tumor-associated antigen, a viral antigen, a bacterial antigen, a parasitic antigen, a mycologic antigen, a prion antigen, an allergen, an alloantigen and an auto-antigen.

22. A pharmaceutical composition for modulating an immune response in a host subject, wherein said composition comprises a non autologous antigen presenting and/or immunomodulatory system and a pharmaceutically acceptable excipient or diluent, wherein said system causes in vitro an increase or a reduction in the host subject T cell response at least 5 times greater than autologous antigen presenting cells from said subject, preferably at least 10 times, 20 times, 50 times or, even more preferably, at least 100 times, and wherein said system shares at least one MHC haplotype with said subject.

23. The composition of claim 22, wherein the antigen presenting and/or immunomodulatory system is selected by a method according to anyone of claims 1 to 21.

24. The composition of claim 22 or 23, wherein the non autologous antigen presenting and/or immunomodulatory system is an allogeneic antigen presenting cell.

25. The composition of anyone of claims 22 to 24, further comprising a selected antigen.

26. The composition of claim 24 or 25, wherein said composition comprises at least two antigen presenting and/or immunomodulatory cells from distinct donors, for combined, separate or sequential administration.

27. The composition of claim 26, wherein the at least two antigen presenting and/or immunomodulatory cells from distinct donors are loaded with the same or distinct antigens.

28. The composition of claim 25, wherein said composition comprises at least two distinct antigens, for combined, separate or sequential administration.

29. A pharmaceutical composition for modulating an immune response in a host subject, wherein said composition comprises an immune cell activated by a non autologous antigen presenting and/or immunomodulatory system selected by a method according to anyone of claims 1 to 21 and a pharmaceutically acceptable excipient or diluent.

30. Use of a non autologous antigen presenting and/or immunomodulatory system selected by a method according to anyone of claims 1 to 21, for the preparation of a pharmaceutical composition for the modulation of an immune response in a subject, wherein said system causes in vitro an increase or a reduction in the subject T cell response at least 5 times greater than autologous antigen presenting cells from the subject, preferably at least 10 times, 20 times, 50 times or, even more preferably, at least 100 times, and wherein said system shares at least one MHC haplotype with said subject.

31. Use of an immune cell activated by a non autologous antigen presenting and/or immunomodulatory system selected by a method according to anyone of claims 1 to 21, for the preparation of a pharmaceutical composition for the modulation of an immune response in a subject.

32. A method for preparing a composition for modulating an immune response in a subject, the method comprising:

(a) providing a library of non autologous antigen presenting and/or immunomodulatory systems,
(b) assessing the antigen presentation and/or immunomodulatory capacity of systems from the library towards white blood cells from the subject,
(c) selecting systems from said library having a significant difference in antigen presentation and/or immunomodulatory capacity for said host subject as compared to autologous antigen presenting cells from said host subject, and
(d) preparing a composition using systems selected in step (c).

33. A bank, wherein the bank comprises a plurality of non-autologous, allele-phenotyped antigen presenting and/or immunomodulatory systems, preferably antigen presenting cells, each of which being comprised in at least one distinct system or cell storage unit for storage of cellular material.

34. A bank according to claim 33, wherein the non autologous antigen presenting and/or immunomodulatory systems comprise antigen presenting cells from different human donors.

35. A method for optimizing an antigen preparation by comparing an immune response to said antigen preparation and to one or several variant antigen preparations thereof, wherein the immune response is determined by contacting said antigen preparations with at least one non autologous antigen presenting and/or immunomodulatory system selected by a method according to anyone of claims 1 to 21 or from a bank according to claim 33 or 34, and selecting variant antigen preparation(s) causing an optimized immune response.

36. A method according to claim 35, wherein the antigen preparations are contacted with at least two antigen presenting cells having a significant difference in antigen presentation capacity.

37. A method according to claim 35 or 36, wherein the antigen preparations are contacted with said presentation systems in the presence of T cells and wherein the antigen specific cytotoxic T cell response is determined.

38. A method for optimizing an antigen preparation by comparing an immune response to said antigen preparation and to one or several variant antigen preparations thereof, wherein the immune response is determined by contacting said antigen preparations with at least two antigen presenting cells having a significant difference in antigen presentation capacity, in the presence of T cells, and selecting variant antigen preparation(s) causing an optimized T cell response.

39. A method according to anyone of claims 35 to 38, wherein the variant antigen preparations comprise a different antigen sequence or formulation, such as a mutated or truncated antigen sequence, a glycosylated antigen, a composition comprising the antigen and any other compound such as a compound exhibiting a different biological activity, an adjuvant, any impurity, or a mixture thereof.

40. A method for selecting a host subject that responds to an immunotherapeutic treatment, comprising a step of contacting immune cells from said subject, in the presence of said immunotherapeutic treatment, with at least one non autologous antigen presenting and/or immunomodulatory system selected by a method according to anyone of claims 1 to 21, and assessing the response of said immune cells, said response being indicative of a responder subject.

41. A method according to claim 40, wherein the immune cells are contacted with at least two antigen presenting cells having a significant difference in antigen presentation capacity.

42. A method according to claim 40 or 41, wherein the immune cells are T cells and wherein an antigen specific cytotoxic T cell response is assessed.

43. A method for selecting a host subject that responds to an immunotherapeutic treatment, comprising a step of contacting T cells from said subject, in the presence of said immunotherapeutic treatment, with at least two antigen presenting cells having a significant difference in antigen presentation capacity, and assessing the response of said T cells, said response being indicative of a responder subject.

**Effector cell proliferation**

**CD8 positive T cell frequency**

**Mart-1 CD8 positive T cell frequency**  **Mart-1 CD8 positive T cell number**

**Figure 1**

**Figure 2**

Figure 3

**Figure 4 A**

**Figure 4 B**

**Figure 5 A**

**Figure 5 B**

NK cell prolifération (x10⁶ cells)

NK cell frequency (%)

NK cells number (LU/culture)

Figure 6

**Figure 7**

Figure 8

**European Patent Office**

## PARTIAL EUROPEAN SEARCH REPORT

Application Number

which under Rule 45 of the European Patent Convention shall be considered, for the purposes of subsequent proceedings, as the European search report

EP 05 29 0815

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 99/47687 A (GENZYME CORPORATION; KAPLAN, JOHANNE; SCARIA, ABRAHAM) 23 September 1999 (1999-09-23) * page 34 * | 1-22,24, 25,33, 34,38-45 | C12Q1/00 G01N33/50 A61K45/00 |
| X | WO 02/074338 A (GSF-FORSCHUNGSGESELLSCHAFT FUER UMWELT UND GESUNDHEIT GMBH; SCHENDEL,) 26 September 2002 (2002-09-26) * pages 5-7,16 * | 22, 24-28, 33,34 | |
| X | WO 2004/002415 A (DANA-FARBER CANCER INSTITUTE, INC; RUPRECHT, RUTH, M; JIANG, SHISONG) 8 January 2004 (2004-01-08) * example 1 * | 33,34 | |
| X | WO 03/103707 A (IMMUNICUM AB; KARLSSON-PARRA, ALEX; WALLGREN, ANNACARIN; ANDERSSON, BE) 18 December 2003 (2003-12-18) * abstract * | 22 | |

-/--

| TECHNICAL FIELDS SEARCHED (Int.Cl.7) |
|---|
| C12Q G01N A61K |

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 16 September 2005 | Knudsen, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**PARTIAL EUROPEAN SEARCH REPORT**

Application Number

EP 05 29 0815

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | PESHWA MADHUSUDAN V ET AL: "Generation of primary peptide-specific CD8+ cytotoxic T-lymphocytes in vitro using allogeneic dendritic cells" CELL TRANSPLANTATION, ELSEVIER SCIENCE, US, vol. 7, no. 1, January 1998 (1998-01), pages 1-9, XP002195712 ISSN: 0963-6897 * page 4; table 1 * | 22 | |
| A | SIMOVA J ET AL: "Immunotherapeutic efficacy of vaccines generated by fusion of dendritic cells and HPV16-associated tumour cells" FOLIA BIOLOGICA (PRAGUE), vol. 51, no. 1, 2005, pages 19-24, XP009053964 ISSN: 0015-5500 * page 22, left-hand column; figure 4 * | 1,22 | TECHNICAL FIELDS SEARCHED (Int.Cl.7) |
| A | US 5 728 378 A (HELLSTRAND ET AL) 17 March 1998 (1998-03-17) * column 6, paragraph 2; table 2 * | 1 | |
| A | US 5 962 320 A (ROBINSON ET AL) 5 October 1999 (1999-10-05) * abstract * | 33,34 | |

-/--

EPO FORM 1503 03.82 (P04C10)

European Patent
Office

**PARTIAL EUROPEAN SEARCH REPORT**

Application Number

EP 05 29 0815

| DOCUMENTS CONSIDERED TO BE RELEVANT | | | CLASSIFICATION OF THE APPLICATION (Int.Cl.7) |
|---|---|---|---|
| Category | Citation of document with indication, where appropriate, of relevant passages | Relevant to claim | |
| A | HUS IWONA ET AL: "The first experience with allogeneic dendritic cells vaccines in ZAP-70 positive and negative untreated B-CLL in early stages of disease." BLOOD, vol. 102, no. 11, 16 November 2003 (2003-11-16), page 435a, XP009054045 & 45TH ANNUAL MEETING OF THE AMERICAN SOCIETY OF HEMATOLOGY; SAN DIEGO, CA, USA; DECEMBER 06-09, 2003 ISSN: 0006-4971 * abstract * | 22 | |
| | ----- | | **TECHNICAL FIELDS SEARCHED** (Int.Cl.7) |

EPO FORM 1503 03.82 (P04C10)

European Patent
Office

INCOMPLETE SEARCH
SHEET C

Application Number

EP 05 29 0815

Claim(s) not searched:
    23,29-32,35-37

Reason for the limitation of the search:

The "non-autologous antigen presenting and/or immunomodulatory system" of claims 23, 29-31, 35-37 is defined as being selected by the method of anyone of claims 1-21. However, the selection by the method of claim 1 does not attribute any special technical feature to the non-autologous antigen presenting and/or immunomodulatory system and claims 23, 29-31 and 35-37 are therefore unclear (Article 84 EPC) and not searchable.

The method of claim 32 is defined as a method of preparing a composition containing a non-autologous antigen presenting and/or immunomodulatory system. However, the steps of the method concern the selection of the system which does not define the method of preparing the composition and does not attribute any special characteristics to the system. Thus, claim 32 lacks clarity (Article 84 EPC) and does not contain any searchable features.

**ANNEX TO THE EUROPEAN SEARCH REPORT
ON EUROPEAN PATENT APPLICATION NO.**                    EP 05 29 0815

This annex lists the patent family members relating to the patent documents cited in the above-mentioned European search report.
The members are as contained in the European Patent Office EDP file on
The European Patent Office is in no way liable for these particulars which are merely given for the purpose of information.

16-09-2005

| Patent document cited in search report | | Publication date | Patent family member(s) | | Publication date |
|---|---|---|---|---|---|
| WO 9947687 | A | 23-09-1999 | AU | 3102799 A | 11-10-1999 |
| | | | CA | 2322660 A1 | 23-09-1999 |
| | | | EP | 1064390 A1 | 03-01-2001 |
| | | | JP | 2002506886 T | 05-03-2002 |
| WO 02074338 | A | 26-09-2002 | DE | 10112851 C1 | 10-10-2002 |
| | | | EP | 1372723 A1 | 02-01-2004 |
| | | | JP | 2004526731 T | 02-09-2004 |
| WO 2004002415 | A | 08-01-2004 | AU | 2003245729 A1 | 19-01-2004 |
| WO 03103707 | A | 18-12-2003 | AU | 2003241252 A1 | 22-12-2003 |
| | | | EP | 1509244 A1 | 02-03-2005 |
| US 5728378 | A | 17-03-1998 | AT | 192338 T | 15-05-2000 |
| | | | AU | 672610 B2 | 10-10-1996 |
| | | | AU | 4366093 A | 30-12-1993 |
| | | | CA | 2136952 A1 | 09-12-1993 |
| | | | DE | 69328558 D1 | 08-06-2000 |
| | | | DE | 69328558 T2 | 04-01-2001 |
| | | | DK | 652768 T3 | 07-08-2000 |
| | | | EP | 0652768 A1 | 17-05-1995 |
| | | | ES | 2147758 T3 | 01-10-2000 |
| | | | GR | 3033894 T3 | 30-11-2000 |
| | | | JP | 2888259 B2 | 10-05-1999 |
| | | | JP | 8502024 T | 05-03-1996 |
| | | | PT | 652768 T | 29-09-2000 |
| | | | SE | 513429 C2 | 11-09-2000 |
| | | | SE | 9201719 A | 04-12-1993 |
| | | | WO | 9324144 A1 | 09-12-1993 |
| US 5962320 | A | 05-10-1999 | AU | 6816194 A | 08-11-1994 |
| | | | CA | 2161095 A1 | 27-10-1994 |
| | | | CN | 1128043 A | 31-07-1996 |
| | | | EP | 0695347 A1 | 07-02-1996 |
| | | | JP | 8509606 T | 15-10-1996 |
| | | | WO | 9424267 A1 | 27-10-1994 |
| | | | US | 5738852 A | 14-04-1998 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3970518 A
- US 4710472 A
- US 6872567 B
- WO 9942128 A

**Non-patent literature cited in the description**

- **BHARDWAJ et al.** *J. Clin. Invest.,* vol. 199 (104), 173-80
- **SCHULTZE JL et al.** *Trends Immunol.,* December 2004, vol. 25 (12), 659-64
- **BANCHEREAU J et al.** *Ann N Y Acad. Sci.,* April 2003, vol. 987, 180-7
- **BERNAN et al.** *J. Immunol.,* 1987, vol. 138, 2100-03
- **GROENEVELD et al.** *Journal of the International Federation of Clinical Chemistry,* 1994, vol. 6, 84-94
- **CLOUGH ; ROTH.** *JAVMA,* 1995, vol. 206, 1208-1216
- **CONSTANTIN CM.** *Biol Res Nurs.,* October 2002, vol. 4 (2), 115-27
- **JEROME KR.** *Apoptosis,* December 2003, vol. 8 (6), 563-71
- **MORETTA A.** *Immunol Today.,* May 2000, vol. 21 (5), 228-34
- **CAMPBELL JD.** *Methods,* October 2003, vol. 31 (2), 150-9
- **EGNER W.** *Adv Exp Med Biol.,* 1993, vol. 329, 263-8
- **CORONEL A.** *Br J Haematol.,* September 2001, vol. 114 (3), 671-80
- **KUBBIES M.** *J Cell Physiol.,* November 1985, vol. 125 (2), 229-34
- **PUTZ T.** *J Clin Immunol.,* November 2004, vol. 24 (6), 653-63